# EUROPEAN PATENT APPLICATION

(11) **EP 3 088 399 A1**
(43) Date of publication of application: **02.11.2016**
(21) Application number: 14873158.1
(22) Date of filing: 18.12.2014
(51) Int. Cl.: C07D 471/04, A61K 31/4375, A61K 31/496, A61K 31/5383, A61K 33/06, A61P 31/04, A61P 43/00, C07D 498/06, C07B 51/00, C07B 61/00

(54) **ALKOXYCARBONYL HEMIACETAL-TYPE ESTER PRODRUG OF PYRIDONE CARBOXYLIC ACID ANTIBACTERIAL DRUG**

(30) Priority: 26.12.2013 JP 2013269911
(71) Applicant: Kinki University, Higashiosaka-shi, Osaka 577-8502 (JP); Educational Corporation Mukogawa Gakuin, Nishinomiya-shi, Hyogo 663-8558 (JP); Nipro Corporation, Osaka-shi, Osaka 531-8510 (JP)
(72) Inventor: MATSUYAMA, Kenji, Higashiosaka-shi Osaka 577-8502 (JP); OTORI, Toru, Higashiosaka-shi Osaka 577-8502 (JP); KIMACHI, Tetsutaro, Nishinomiya-shi Hyogo 663-8179 (JP)
(74) Representative: dompatent von Kreisler Selting Werner - Partnerschaft von Patent- und Rechtsanwälten mbB
(86) International application number: PCT/JP2014/083554
(87) International publication number: WO 2015/098693

(57) **Abstract**

The present invention provides a hemiacetal ester prodrug of the pyridone carboxylic acid antibacterial drug, wherein the prodrug is characterized in that, (i) it is hydrolyzed promptly after intestinal absorption and regenerates the pyridone carboxylic acid antibacterial drug as a drug substance, (ii) a one-dose package is possible by preventing chelate-formation in intestines in an oral administration, and (iii) the normal bacterial flora of the intestinal bacterial flora is not influenced at all until the ester is absorbed, because the hemiacetal-type esterification is executed on the carboxylic acid which is a center of the antibacterial action, resulting in prevention of the pseudomembranous colitis. The prodrug is an alkoxy carbonyl hemiacetal-type ester represented by the chemical formula (I), which is useful as a prodrug for inhibiting the enteric-bacterium-shift side effect, and can be used in combination with polyvalent metal-containing medicines.

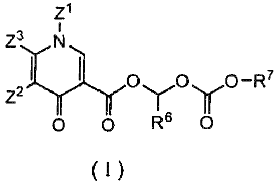

## Description

### TECHNICAL FIELD

The present invention relates to a hemiacetal ester prodrug of a pyridone carboxylic acid antibacterial drug. Since the ester prodrug does not form an insoluble chelate when co-administered with a metal-containing drug, it can be used together with a metal-containing drug simultaneously (that is; a one-dose package is possible). And the ester prodrug does not cause microbial substitution in the intestinal environment. That is, since antibacterial action of the ester prodrug is not exerted to the normal bacterial flora in intestines, it does not have side effects, such as pseudomembranous colitis caused by Clostridium difficile ordinarily suppressed by the normal bacterial flora.

### BACKGROUND ART

The pyridone carboxylic acid antibacterial drug is a synthetic antibacterial drug which has a pyridone carboxylic acid framework represented by the chemical formula (I'): 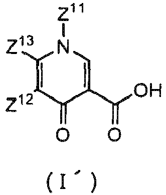 , wherein Z¹¹-Z¹³ represent hydrocarbons, nitrogen-containing hydrocarbons, or oxygen-containing hydrocarbons optionally substituted, and those neighboring two may configure a ring. As pyridone carboxylic acid antibacterial drugs, known are the nalidixic acid (represented by the chemical formula (1)) which has an outstanding bactericidal action to gram negatives (especially, colibacillus, pneumobacillus, dysentery bacillus, etc.) and the pipemidic acid (represented by the chemical formula (2)) which has a still higher antimicrobial action to a gram negative and is effective also in the pseudomonas aeruginosa etc. These nalidixic acid and pipemidic acid are also referred to as a "quinolone antibacterial drug" hereinafter.

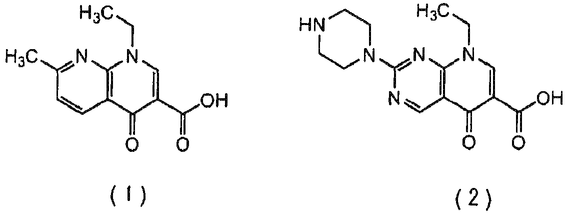

Moreover, by introducing fluorine atom(s) into the framework of these quinolone antibacterial drugs, the antimicrobial activity to the whole gram negatives comprising pseudomonas aeruginosa increases, and the antimicrobial spectrum also expands. As a result, the antimicrobial activity to gram positives, such as staphylococcus aureus, streptococcus pyogenes and enterococcus, is also exerted. These fluorine containing quinolone antibacterial drugs are also called "new quinolones." These quinolone families and new quinolones are named generically as "pyridone carboxylic acid antibacterial drugs." Moreover, quinolone antibacterial drugs may be referred to as "quinolone carboxylic acids", and new quinolones may be referred to as "new quinolone carboxylic acids" hereinafter. The pyridone carboxylic acid antibacterial drugs are antibacterial drugs in which the antibacterial action is exerted by inhibiting DNA gyrase and inhibiting replication of DNA. At this point, the pyridone carboxylic acid antibacterial drugs are different from the ordinal cephem or penicillin antibiotics (the action mechanism of antibiotics is based on the cell-wall-synthesis inhibition), or the macrolide type antibiotics (the action mechanism of macrolides is based on the protein synthesis inhibition). And in the pyridone carboxylic acid antibacterial drugs, a broad antimicrobial spectrum is exerted not only to the above-mentioned gram positives and negative bacillus but also to the mycoplasmas which do not have a cell wall (Jun-ichi Mitsuyama, Folia Pharmacol. Jpn. 130,287-293 (2007)). Moreover, especially new quinolones are the first-choice medicine in intractable urinary tract infection in the urology department field because of their excellent kidney migration (Sohichi Arakawa, medicament news, 1815, 4-7 (2004)).

As a typical example of the new quinolone (also referred to as "NQ type") antibacterial drugs, a substituted type is mentioned wherein the 6th position is substituted by a fluorine atom and the 7th position is substituted by an amino-containing group in the quinolone carboxylic acid represented by the chemical formula (A): 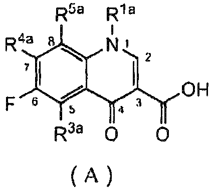
. wherein R^{1a} represents the following chemical formula: 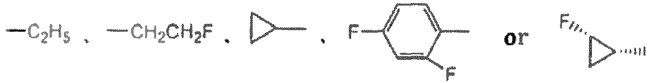
, R^{3a} represents -H, -NH₂ or -CH₃,
, R^{4a} represents the following chemical formula: 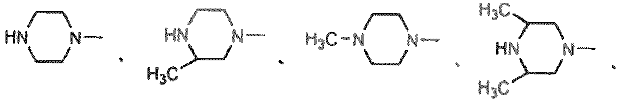 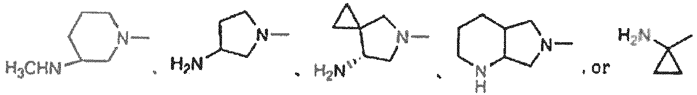
, and R^{5a} represents -H, -F, -Cl, or OCH₃. The typical new quinolones belonging to this type are represented in Table 1:

**Table 1. Examples of NQ antibacterial drugs**

| 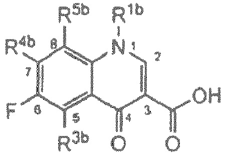 | | | | |
|---|---|---|---|---|
| | R^{1b} | R^{3b} | R^{4b} | R^{5b} |
| Norfloxacin | C₂H₅ | H | 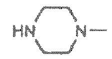 | H |
| Lomefloxacin | C₂H₅ | H | 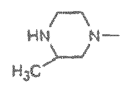 | F |
| Fleroxacin | CH₂CH₂F | H | 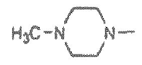 | F |
| Ciprofloxacin | 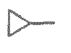 | H | 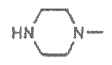 | H |
| Sparfloxacin | 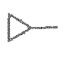 | NH₂ | 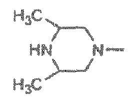 | F |
| Grepafloxacin | 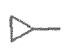 | CH₃ | 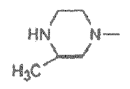 | H |
| Balofloxacin | 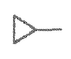 | H | 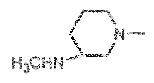 | OCH₃ |
| Temafloxacin | 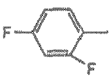 | H | 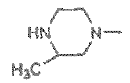 | H |
| Sitafloxacin | 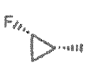 | H | 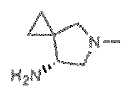 | Cl |
| Moxifloxacin | 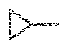 | H | 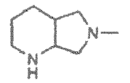 | OCH₃ |

Furthermore, new quinolone antibacterial drugs represented by the chemical formula (B): 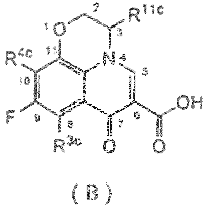 are superior both in the antibacterial action and the antibacterial spectrum, wherein the chemical formula (B) has an oxazine ring formed by ring formation of R^{1a} of 1st position and R^{5a} of 3rd position together with N-atom and C-atom of the quinolone ring in the chemical formula (A). The typical examples of the chemical formula (B) are represented in Table 2:

**Table 2. Examples of NQ antibacterial drugs**

| 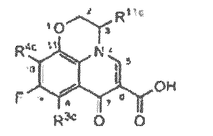 | | | |
|---|---|---|---|
| | R^{11c} | R^{3c} | R^{4c} |
| Ofloxacin | 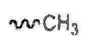 | H | 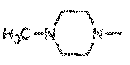 |
| Levofloxacin | 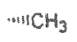 | H | 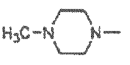 |
| Pazufloxacin | 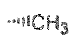 | H | 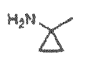 |

Furthermore, new quinolones of a naphthyridine type are also used widely. The typical examples of the naphthyridine type are represented in Table 3:

**Table 3. Examples of NQ antibacterial drugs: Naphthyridine type**

| 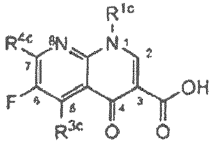 | | | |
|---|---|---|---|
| | R^{1o} | R^{3o} | R^{4o} |
| Enoxacin | C₂H₅ | H | 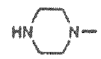 |
| Tosufloxacin | 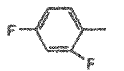 | H | 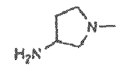 |

Among typical new quinolones, the most notable one is Levofloxacin (often briefly written as LVFX hereinafter) in Table 2. The LVFX is one of the most frequently-used new quinolone (NQ)-type antibacterial drug all over the world. Levofloxacin is a chemical species taken out in a high purity as only 3S form which is an active core among two optical isomers of Ofloxacin (Table 2) developed as a racemic form:
(3S:(3S)-9-fluoro-3-methyl-10-(4-methyl-1-piperazinyl)-7-oxo-2,3-dihydro-7H-pyrid [1,2,3-de] [1,4] benzoxazine-6-carboxylic acid). The antibacterial action and the safety became almost twice as high as the racemic form. That is, it is demonstrated clearly that 3S-form is an effective form and 3R-form is a toxic form. (Bernard Rouveix, Antibacterial Safety Assessment, International Journal of Antimicrobial Agents 21, 215-221, (2003)).

Ester derivatives described in JP patent No. 4669607 (equivalent international publication: WO1999/014214) are mentioned as ester derivatives of new quinolone. This reference describes an invention concerning novel new quinolones represented by the following chemical formula (D): 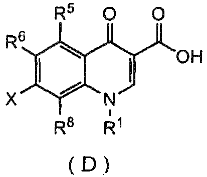 (with regard to the symbols in the chemical formula (D), refer to the reference.), which is different from the conventional type. As examples of such derivatives, "biohydrolyzable esters" are mentioned. In paragraph "0055" of the description of the reference, the following descriptions are found:
""Biohydrolyzable esters" are esters of the compounds of the invention, wherein the esters do not essentially interfere, preferably do not interfere, with the antimicrobial activity of the compounds, or wherein the esters undergo rapid hydrolysis in vivo yielding an active parent drug. Many such esters are known in the art, as described in U.S. Patent No. 4,783,443, issued by Johnston and Mobashery on November 8, 1988 (incorporated by reference herein). Such esters include lower alkyl esters, lower acyloxy-alkyl esters (such as acetoxymethyl, acetoxyethyl, aminocarbonyloxymethyl, pivaloyloxymethyl and pivaloyloxyethyl esters), lactonyl esters (such as phthalidyl and thiophthalidyl esters), lower alkoxyacyloxyalkyl esters (such as methoxycarbonyloxymethyl, ethoxycarbonyloxyethyl and isopropoxycarbonyloxyethyl esters), alkoxyalkyl esters, choline esters and alkylacylaminoalkyl esters (such as acetamidomethyl esters)." However, nomination of any specified compound name can't be found for the "biohydrolyzable esters" at all.

In addition, as lower alkoxy carbonyl hemiacetal-type ester derivatives of the antibacterial drug except for pyridone carboxylic acid, disclosed are the following examples (E) and (F):

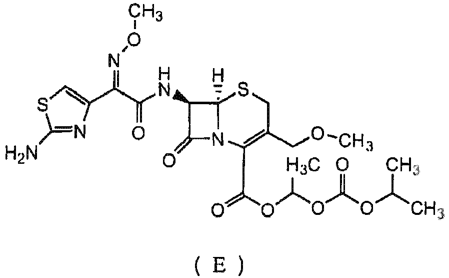

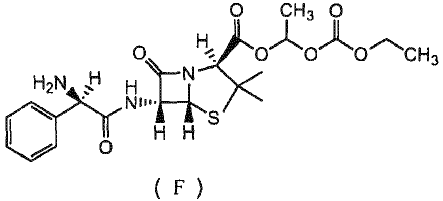

However, no alkoxy carbonyl hemiacetal-type ester derivative of the pyridone carboxylic acid antibacterial drugs (quinolone and new quinolone antibacterial drugs) has been known until now.

### PRIOR ART DOCUMENTS

### PATENT DOCUMENTS

### (1) JP Patent No. 4669607 (equivalent international publication: WO1999/014214)

### NON-PATENT DOCUMENTS

(1) Jyunichi Mitsuyama, Folia Pharmacol. Jpn. 130,287-293 (2007)
(2) Souichi Arakawa, medicament news, 1815,4-7 (2004)
(3) Bernard Rouveix, Antibacterial Safety Assessment, International Journal of Antimicrobial Agents 21, 215-221 (2003).

### DISCLOSURE OF INVENTION

### PROBLEMS TO BE SOLVED BY THE INVENTION

For the pyridone carboxylic acid antibacterial drugs, insolubilization by chelate formation in co-administration of a polyvalent metal-containing medicine is described in the paragraph of concomitant use cautions of a package insert. That is, there is a description that administration of a metal-containing drug should be delayed for 2 hours or more after administration of the pyridone carboxylic acid antibacterial drug, because absorption of the pyridone carboxylic acid antibacterial drug will fall down due to insoluble chelation with metal-containing drug and its effect will decrease when simultaneous concomitant use of the metal-containing drug is carried out. The concomitant use cautions are matters which obstruct the one-dose package frequently used in recent years along with the aging, and cause a problem. Moreover, the antibacterial drugs cause the microbial substitution to Clostridium difficile based on the bactericidal effect exerted on the intestinal bacterial flora until the drugs are absorbed, and tend to cause the pseudomembranous Colitis by the microbial substitution. These problems are described in a package insert by the 2nd of the serious side effects (in the case of Ciprofloxacin), and poses a problem of clinical. However, neither of the problems has still been solved in spite of years of trials to solve these problems.

Moreover, in those trials, the prior art is hardly found for trying to solve these problems by prodrug derivatization through esterification etc.

The subject of the present invention is solving the above-mentioned problems. That is, the prevention of chelate forming-capability of the pyridone carboxylic acid antibacterial drug, and the prevention from side effects of pseudomembranous colitis etc. by the enteric-bacterium shift phenomenon are subject matters of the present invention. Here, the pyridone carboxylic acid antibacterial drug is a general term for the quinolone antibacterial drug and the new quinolone antibacterial drug which have a pyridone carboxylic acid framework as a core structure.

It is known that, when the pyridone carboxylic acid antibacterial drug is used together with a polyvalent metal-containing medicine, for example, polyvalent metal-containing medicine (Mg²⁺ or Al³⁺ containing drug) or an iron drug, an insoluble chelate, for example, the chelate having the following chemical formula (G): 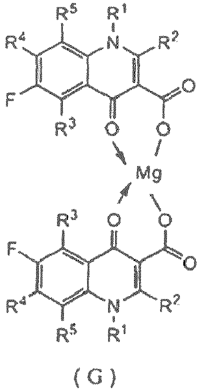 is formed between the metal ion and the carboxyl group of the 3rd position (the 6th position in the case of oxazine-containing NQ antibacterial drug) and the carbonyl group of the 4th position (the 7th position in the case of oxazine-containing NQ antibacterial drug) of the pyridone carboxylic acid antibacterial drug, and as a result, the absorption into the body is inhibited.

Accordingly, when using these metal-containing agents together with the pyridone carboxylic acid antibacterial drug, it is necessary to take 2 to 3 hours interval for dosing, and as a result, this interval has become a neck upon administration. In a package-insert of 2013 edition (DAIICHI SANKYO) of "Cravit tablet 250 mg / Cravit tablet 500 mg / Cravit fine granule 10%" which are Levofloxacin medicines, and in the 2nd part of the interactions and concomitant use cautions, notes are described that "A chelate is formed according to the concomitant use of aluminum etc., and, therefore, the effects of these medicines tend to decrease. Accordingly administration of the aluminum agent for patients should be after 1-2 hours from the administration of these medicines." Refer to http://www.info.pmda.go.jp/go/pack/6241013C2024_1_09/.

Similarly, pseudomembranous colitis by the enteric-bacterium shift phenomenon in oral administration of the pyridone carboxylic acid antibacterial drug is a serious side effect associated with its administration, and the prevention of this side effect is a big challenge. (Pseudomembranous colitis is described as the 9th of the serious side effects in the package-insert 2013 edition (DAIICHI SANKYO) of "Cravit tablet 250 mg / Cravit tablet 500 mg / Cravit fine granule 10%" which are Levofloxacin medicines.)

In the patent document 1 (JP 4669607B (equivalent to WO1999/014214)) regarding the above-mentioned novel new quinolones, there is no description for preventing a chelate formation and an enteric-bacterium shift phenomenon in oral administration as a subject matter of the invention. Moreover, various ester derivatives described in the description in paragraph "0055" of the document are mere illustrative listings. Furthermore, there is no description for suggesting a positive use of the ester derivative as a prodrug. In addition, all working examples are of carboxylic types and there is no working example which actually supports the ester form.

That is, as mentioned above, it is pointed out that, in the case of oral administration of the pyridone carboxylic acid antibacterial drug, the microbial substitution occurs in intestines by the strong bactericidal action of the pyridone carboxylic acid antibacterial drug, and Clostridium difficile which is a kind of normal bacterial flora in intestines and has resistance to many antibiotics increases, and then, the toxins produced by the Clostridium difficile stimulate the large intestine and cause pseudomembranous colitis. (Kentaro Oka: Probiotics for Clostridium difficile-associated disease; Journal of intestinal microbiology, volume 27, No. 2, p86, 2013).

In Banan (registered trademark) (generic name "cefpodoxime proxetil") of the oral cephem antibiotic and Pengood (registered trademark) of the synthetic penicillin type preparation (generic name "bacampicillin hydrochloride"), with regard to the above-mentioned isopropoxycarbonyl hemiacetal-type ester derivative (E) and the carboethoxy hemiacetal-type ester derivative (F), a purpose of prodrug derivatization is an improvement of the oral absorption by raising lipophilicity, but is not aiming at prevention of a chelate formation or an enteric-bacterium shift side effect in oral administration, whereas the latter two are the subject matters of the present invention.

Accordingly, the subject to be solved is different from those of the present invention.

The present inventors examined extensively prodrug derivatizations of the pyridone carboxylic acid antibacterial drug for preventing the chelate formation and the enteric-bacterium shift side effect in oral administration.

### MEANS FOR SOLVING THE PROBLEM

At the beginning, the present inventors examined a prodrug derivatization by esterification of a pyridone carboxylic group of the pyridone carboxylic acid antibacterial drug, in which the carboxylic group is a main group of the insoluble chelate formation and also is an active center for sterilizing Enterobacterium. However, as will be explained later by comparative examples, in alkyl esterification such as methyl-, ethyl-, isopropyl-esters, etc. (also referred to as "simple esterification"), the blood concentration of the pyridone carboxylic acid antibacterial drug by the intestinal absorption was not observed at all. Therefore, the simple ester was turned out not to work as a prodrug. That is, it turned out that the pyridone carboxylic acid antibacterial drug as a drug substance is not regenerated in blood because the simple ester derivative is not absorbed by the intestinal tract, or, even if absorbed, the simple ester is not hydrolyzed by the intestinal tract.

Then, as a result of further extensive studies by carrying out an alkoxy carbonyl hemiacetal-type esterification of the carboxyl group of the 3rd position of the pyridone carboxylic acid antibacterial drug (hereinafter, simply called "hemiacetal-type esterification"), the present inventors surprisingly found that: (i) after the ester is absorbed in the intestinal tract, the ester is hydrolyzed promptly to regenerate the pyridone carboxylic acid antibacterial drug of the active ingredient, (ii) a one-dose package is possible by preventing chelate formation in intestines in the oral administration, and (iii) the normal bacterial flora of the intestinal bacterial flora is not influenced at all until the ester is absorbed because the hemiacetal-type esterification is carried out on the carboxylic acid group which is the center of the antibacterial action, therefore, pseudomembranous colitis is hard to be caused. Accordingly, the desired prodrug of the pyridone carboxylic acid antibacterial drug of the present invention has been attained.

Specifically, the present invention relates to the following subject matters: (i) an alkoxy carbonyl hemiacetal-type ester prodrug of the pyridone carboxylic acid antibacterial drug (especially, an alkoxy carbonyl hemiacetal-type ester prodrug of the new quinolone carboxylic acid antibacterial drug for prevention of the enteric-bacterium shift side effect and/or for prevention of the chelate formation with the polyvalent metal-containing medicine, (ii) the ester compound itself, (iii) a method for producing the compound, (vi) the antibacterial prodrug composition comprising the ester prodrug, and, (v) a kit consisting of the pharmaceutical composition and a polyvalent metal-containing medicine.

The alkoxy carbonyl hemiacetal-type ester of the carboxylic acid as used in the context of the present invention refers to the ester derivative represented by the following chemical formula: 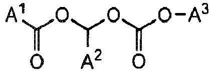 , wherein A¹, A², and A³ represent any hydrocarbon groups or hetero atom-containing hydrocarbon groups optionally substituted. Hereinafter, this "alkoxy carbonyl hemiacetal-type ester" is optionally abbreviated only as "hemiacetal-type ester."

Hereinafter, specified embodiments of the present invention are explained. In the explanation of substituents, the phrase "may be substituted" means that it may be non-substituted or may be substituted by any substituent(s) selected from a hydrocarbon group and a hetero atom-containing group such as oxygen-containing, nitrogen-containing or halogen-containing group.

That is, one embodiment of the present invention is:
An ester prodrug for use for suppression of an enteric-bacterium shift side effect and/or for use in combination with a polyvalent metal-containing medicine, wherein the ester is an alkoxy carbonyl hemiacetal-type ester of a pyridone carboxylic acid antibacterial drug represented by the chemical formula (I): 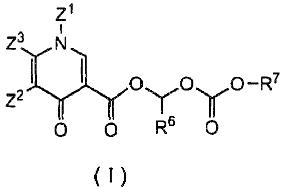
   , wherein Z¹-Z³ are the same or different and each is hydrocarbon, nitrogen-containing hydrocarbon, or oxygen-containing hydrocarbon optionally substituted, wherein those adjacent two of Z¹-Z³ may join together to configure a hydrocarbon ring, nitrogen-containing heterocycle, oxygen-containing heterocycle, or nitrogen and oxygen-containing heterocycle optionally substituted,
   R⁶ represents H or C₁-C₆ alkyl, and
   R⁷ represents C₁-C₆alkyl.

A further embodiment of the present invention is:
The ester prodrug, wherein the enteric bacterium-shift side effect of the pyridone carboxylic acid antibacterial drug is pseudomembranous colitis.

A further embodiment of the present invention is:
The ester prodrug, wherein the polyvalent metal-containing medicine used in combination with an alkoxy carbonyl hemiacetal-type ester of the pyridone carboxylic acid antibacterial drug is a polyvalent metal antacid which can be administered orally and substantially simultaneously with the alkoxy carbonyl hemiacetal-type ester prodrug of the pyridone carboxylic acid antibacterial drug.

A further embodiment of the present invention is:
The ester prodrug, wherein the alkoxy carbonyl hemiacetal-type ester of the pyridone carboxylic acid antibacterial drug is represented by the chemical formula (II): 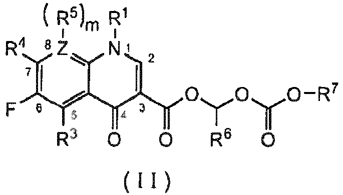 , wherein m represents 1 or 0, R⁶ and R⁷ are the same as described above, Z represents C or N, R¹ represents C₁-C₄ alkyl optionally substituted with halogen, C₃-C₆ cycloalkyl optionally substituted with halogen, or phenyl optionally substituted with halogen, R³ represents H, NH₂, or methyl, R⁴ represents 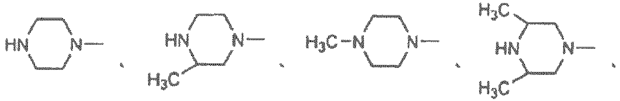 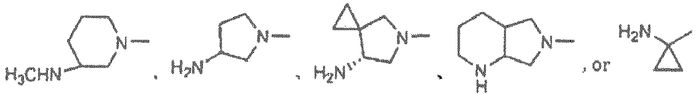 , R⁵ represents H, F, Cl, or OCH₃, or R⁵ and R¹ may join together with N of the 1st position or Z of the 8th position to form nitrogen-containing heterocycle or nitrogen and oxygen-containing heterocycle optionally substituted (provided that, when Z=N, m=0). Z and R¹ - R⁷ are collectively represented in the following table 4:

**Table 4**

| Z | C, N |
|---|---|
| R¹ | C1-C4 Alkyl optionally substituted with halogen C3-C6 Cycloalkyl optionally substituted with halogen, or C6 Aryl opionally substituted with halogen |
| R³ | H, NH₂ or CH₃ |
| R⁴ | 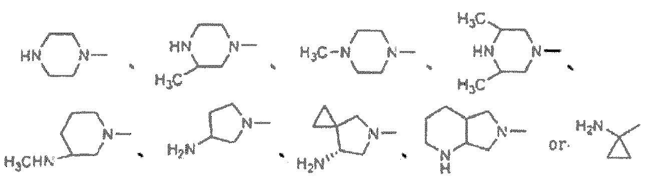 |
| R⁵ | H,F,CL or OCH₃ |
| R⁶ | H or C1-C6 Alkyl |
| R⁷ | C1-C6 Alkyl |

A further embodiment of the present invention is:
The ester prodrug, wherein,
the alkoxy carbonyl hemiacetal-type ester of the pyridone carboxylic acid antibacterial drug of the chemical formula (II) is the alkoxy carbonyl hemiacetal-type ester of the pyridone carboxylic acid antibacterial drug represented by the chemical formula (III): 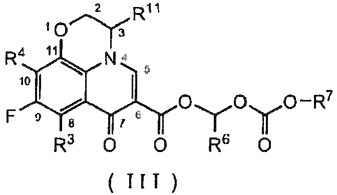
   , wherein R³-R⁴ and R⁶-R⁷ are the same as above, and R¹¹ is:

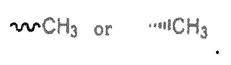

A further embodiment of the present invention is:
The ester prodrug, wherein the ester prodrug corresponds to Z = C and m = 1 in the chemical formula (II) for the alkoxy carbonyl hemiacetal-type ester of the pyridone carboxylic acid antibacterial drug, wherein the ester prodrug is at least one selected from the hemiacetal-type ester derivative of the chemical formula (II-1): 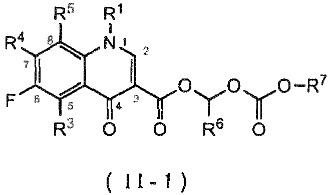
   , wherein which has the combination of each substituent R¹, R³, R⁴, R⁵, R⁶, and R⁷, and is selected from:
      alkoxy carbonyl hemiacetal-type ester of Norfloxacin, wherein R¹ = ethyl, R³= H, R⁴ = 1-piperazinyl, R⁵ = H, R⁶ = H or C₁-C₆ alkyl, and R⁷ = C₁-C₆ alkyl;
      alkoxy carbonyl hemiacetal-type ester of Lomefloxacin, wherein R¹ = ethyl, R³
   = H, R⁴ = 3-methyl-1-piperazinyl, R⁵ = F, R⁶ = H or C₁-C₆ alkyl, and R⁷ =C₁-C₆ alkyl;
      alkoxy carbonyl hemiacetal-type ester of Fleroxacin, wherein R¹ = CH₂CH₂F, R³ = H, R⁴ = 4-methyl-1-piperazinyl, R⁵ = F, R⁶= H or C₁-C₆ alkyl, and R⁷ = C₁-C₆ alkyl;
      alkoxy carbonyl hemiacetal-type ester of Ciprofloxacin, wherein R¹ = cyclopropyl, R³ = H, R⁴ = 1-piperazinyl, R⁵ = H, R⁶= H or C₁-C₆ alkyl, and R⁷ = C₁-C₆ alkyl;
      alkoxy carbonyl hemiacetal-type ester of Sparfloxacin, wherein R¹ = cyclopropyl, R³ = NH₂, R⁴=3, 5- dimethyl- 1-piperazinyl, R⁵ =F, R⁶=H or C₁-C₆ alkyl, and R⁷ =C₁-C₆ alkyl;
      alkoxy carbonyl hemiacetal-type ester of Grepafloxacin, wherein R¹= cyclopropyl, R³=CH₃, R⁴=3- methyl-1-piperazinyl, R³=H, R⁶=H or C₁-C₆ alkyl, and R⁷ =C₁-C₆ alkyl;
      alkoxy carbonyl hemiacetal-type ester of Balofloxacin, wherein R¹ = cyclopropyl, R³=H, R⁴ =3-methylamino-1-piperidino, R⁵=OCH₃, R⁶=H or C₁-C₆ alkyl, and R⁷ =C₁-C₆ alkyl;
      alkoxy carbonyl hemiacetal-type ester of Temafloxacin, wherein R¹=2,4-difluorophenyl, R³=H, R⁴ =3-methyl-1-piperazinyl, R⁵=H, R⁶=H or C₁-C₆ alkyl, and R⁷ =C₁-C₆ alkyl; and
      alkoxy carbonyl hemiacetal-type ester of Sitafloxacin, wherein
         R¹ = 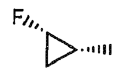
         , R³ = H,
         R⁴ = 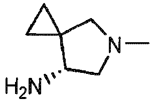
         , R⁵ =Cl, R⁶=H or C₁-C₆ alkyl, and R⁷ =C₁-C₆ alkyl.

These alkoxy carbonyl hemiacetal-type esters are collectively represented in table 5:

**Table 5**

| Hemiacetal type esters of NQ antibacterial drugs | | | | | | |
|---|---|---|---|---|---|---|
| 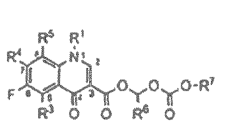 | | | | | | |
| | R¹ | R³ | R⁴ | R⁵ | R⁸ | R⁷ |
| Norfloxacin hemiacetal type ester | C₂H₅ | H | 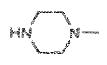 | H | H or C1-C6 alkyl | C1-C6 alkyl |
| Lomefloxacin hemiacetal type ester | C₂H₅ | H | 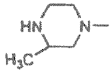 | F | | |
| Fleroxacin hemiacetal type ester | CH₂CH₂F | H | 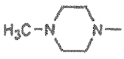 | F | ibid | ibid |
| Ciprofloxacin hemiacetal type ester | 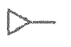 | H | 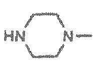 | H | ibid | ibid |
| Sparfloxacin, hemiacetal type ester | 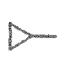 | NH₂ | 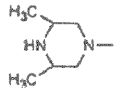 | F | ibid | ibid |
| Grepafloxacin hemiacetal type ester | 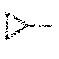 | CH₃ | 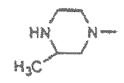 | H | ibid | ibid |
| Balofloxacin hemiacetal type ester | 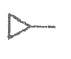 | H | 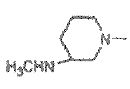 | OCH₃ | ibid | ibid |
| Temafloxacin hemiacetal type ester | 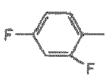 | H | 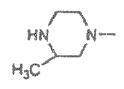 | H | ibid | ibid |
| Sitaflofloxacin hemiacetal type ester | 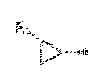 | H | 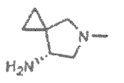 | Cl | ibid | ibid |

A further embodiment of the present invention is:
The ester prodrug, wherein the combination of each substituent R¹¹, R³, R⁴, R⁶, and R⁷ in alkoxy carbonyl hemiacetal-type ester of the pyridone carboxylic acid antibacterial drug represented by the chemical formula (III) is at least one alkoxy carbonyl hemiacetal-type ester of the pyridone carboxylic acid antibacterial drug selected from the followings:
   alkoxy carbonyl hemiacetal-type ester of Ofloxacin, wherein R¹¹ = methyl, R³=H, R⁴ =4-methyl-1-piperazinyl, R⁶=H or C₁-C₆ alkyl, and R⁷ =C₁-C₆ alkyl,
   alkoxy carbonyl hemiacetal-type ester of Levofloxacin, wherein R¹¹= 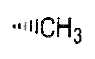
, R³=H, R⁴=4-methyl-1-piperazinyl, R⁶=H or C₁-C₆ alkyl, and R⁷ =C₁-C₆ alkyl, and alkoxy carbonyl hemiacetal-type ester of the Pazufloxacin, wherein R¹¹= 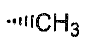
, R³=H, R⁴=1-amino-1-cyclopropyl, R⁶=H or C₁-C₆ alkyl, and R⁷ -C₁-C₆ alkyl.

These alkoxy carbonyl hemiacetal-type esters are collectively represented in table 6:

**Table 6**

| Hemiacetal type esters of oxazine ring-containing NQ antibacterial drugs | | | | | | |
|---|---|---|---|---|---|---|
| 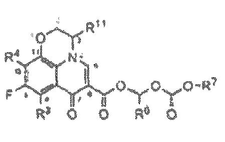 | | | | | | |
| | R¹¹ | R³ | R⁴ | R⁵ | R⁶ | R⁷ |
| Hemiacetal type ester of Ofloxacin | 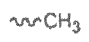 | H | 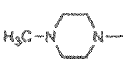 | H | H or C1-C6 alkyl | C1-C6 alkyl |
| Hemiacetal type ester of Levofloxacin | 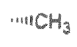 | H | 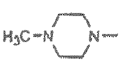 | H | ibid | ibid |
| Hemiacetal type ester of Pazufloxacin | 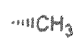 | H | 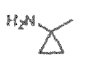 | H | ibid | ibid |

A further embodiment of the present invention is:
The ester prodrug, wherein alkoxy carbonyl hemiacetal-type ester of the pyridone carboxylic acid antibacterial drug of the chemical formula (III) is alkoxy carbonyl hemiacetal-type ester of Levofloxacin represented by the chemical formula (IV): 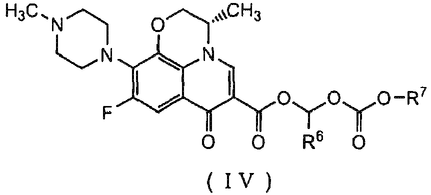 , wherein R⁶ and R⁷ are the same as mentioned as above.

A further embodiment of the present invention is:
The ester prodrug, wherein alkoxy carbonyl hemiacetal-type ester of the pyridone carboxylic acid antibacterial drug of the chemical formula (III) is ethoxycarbonyl-1-ethyl hemiacetal-type ester of Levofloxacin represented by the chemical formula (V): 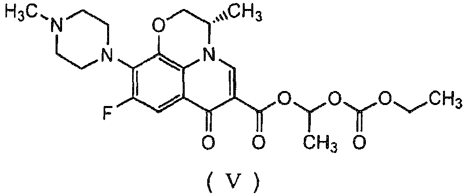 , or isopropoxycarbonyl-1-ethyl hemiacetal-type ester of the Levofloxacin represented by the chemical formula (VI):

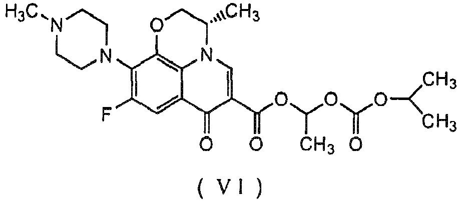

A further embodiment of the present invention is:
The ester prodrug, wherein the ester prodrug corresponds to Z = N and m = 0 in the chemical formula (II) for the alkoxy carbonyl hemiacetal-type ester of the pyridone carboxylic acid antibacterial drug, wherein the ester prodrug is selected from the hemiacetal-type ester derivative of the chemical formula (VII): 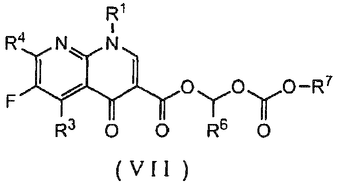 , wherein R¹ = ethyl, R³=H, R⁴=1-piperazinyl, R⁶=H or C₁-C₆ alkyl, and R⁷ =C₁-C₆ alkyl (that is; alkoxy carbonyl hemiacetal-type ester of Enoxacin), or R¹=2,4-difluorophenyl, R³=H, R⁴=3-amino-1-pyrrolidinyl, R⁶=H or C₁-C₆ alkyl, and R⁷=C₁-C₆ alkyl (that is; alkoxy carbonyl hemiacetal-type ester of Tosufloxacin).

A further embodiment of the present invention is:
The ester prodrug, wherein the alkoxy carbonyl hemiacetal-type ester of Enoxacin is ethoxycarbonyl 1-ethyl hemiacetal-type ester of Enoxacin represented by the chemical formula (VIII): 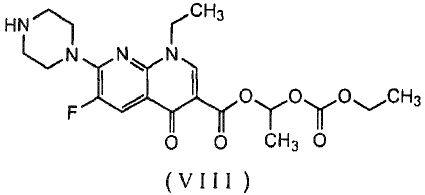 , or isopropoxycarbonyl-1-ethyl hemiacetal-type ester of Enoxacin represented by the chemical formula: (IX):

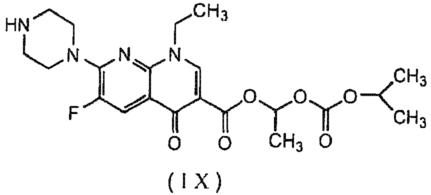

A further embodiment of the present invention is:
The ester prodrug, wherein the alkoxy carbonyl hemiacetal-type ester prodrug of Tosufloxacin is ethoxycarbonyl 1-ethyl hemiacetal-type ester prodrug of Tosufloxacin represented by the chemical formula (X): 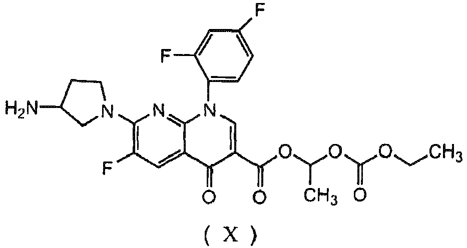 , or isopropoxycarbonyl-1-ethyl hemiacetal-type ester of Tosufloxacin represented by the chemical formula (XI):

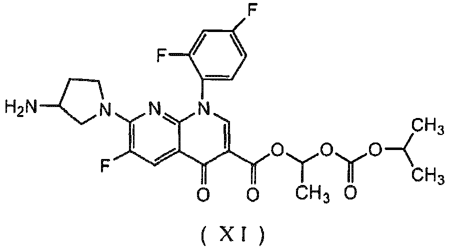

A further embodiment of the present invention is:
The pharmaceutical composition for oral administration comprising alkoxy carbonyl hemiacetal-type ester prodrug of the pyridone carboxylic acid antibacterial drug and excipients.

A further embodiment of the present invention is:
The pharmaceutical composition for administering orally and substantially simultaneously with a polyvalent metal-containing medicine.

A further embodiment of the present invention is:
The pharmaceutical composition, wherein the polyvalent metal-containing medicine is a polyvalent metal-containing antacid.

A further embodiment of the present invention is:
A kit consisting of the pharmaceutical composition and a polyvalent metal-containing medicine.

A further embodiment of the present invention is:
The kit, wherein the polyvalent metal-containing medicine is a polyvalent metal-containing antacid.

A further embodiment of the present invention is:
An alkoxy carbonyl hemiacetal-type ester compound of the pyridone carboxylic acid antibacterial drug, wherein the ester compound is an alkoxy carbonyl hemiacetal-type ester or phamaceutically acceptable salt thereof represented by the chemical formula (I): 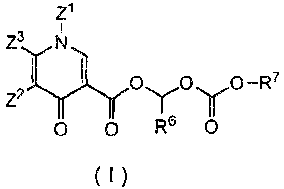 , wherein each of Z¹-Z³ represents hydrocarbon, nitrogen-containing hydrocarbon, or oxygen-containing hydrocarbon optionally substituted, wherein those adjacent two of them may join together to configure a hydrocarbon ring, nitrogen-containing heterocycle, oxygen-containing heterocycle, or nitrogen- and oxygen-containing heterocycle optionally substituted, R⁶ represents H or C₁-C₆ alkyl, and R⁷ represents C₁-C₆ alkyl.

A further embodiment of the present invention is:
The alkoxy carbonyl hemiacetal-type ester compound of the pyridone carboxylic acid antibacterial drug according to [18], wherein the ester compound is represented by the chemical formula (II): 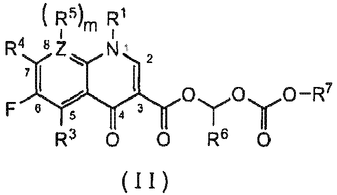
, wherein, m represents 1 or 0, R⁶ and R⁷ are the same as above, Z represents C or N, R¹ represents C₁-C₄ alkyl optionally substituted with halogen, C₃-C₆ cycloalkyl optionally substituted with halogen, or phenyl optionally substituted with halogen, R³ represents H, NH₂, or methyl, R⁴ represents: 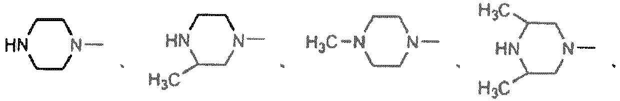 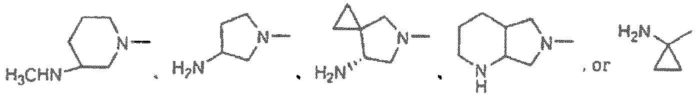
, R⁵ represents H, F, Cl, or OCH₃, or
R⁵ and R¹ may join together with N of the 1st position and Z of the 8th position to configure nitrogen-containing heterocycle, or nitrogen and oxygen-containing heterocycle optionally substituted (provided that; m = 0 when Z = N),
or when Z = C, R¹ and R⁵ join together with N atom and C atom of the quinolone ring to configure a cyclic compound resulting in the formation of alkoxy carbonyl hemiacetal-type ester of an oxazine-containing pyridone carboxylic acid antibacterial drug represented by the chemical formula (III): 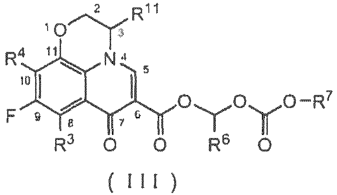
, wherein R³-R⁴ and R⁶-R⁷ are the same as above, and R¹¹ is: 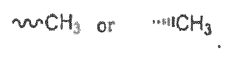
Z and R¹ to R⁷ are collectively represented in the following table 7:

**Table 7**

| Z | C, N |
|---|---|
| R¹ | C1-C4 Alkyl optionally substituted with halogen C3-C6 Cycloalkyl optionally substituted with halogen, or C6 aryl optionally substituted with halogen |
| R³ | H, NH₂ or CH₃ |
| R⁴ | 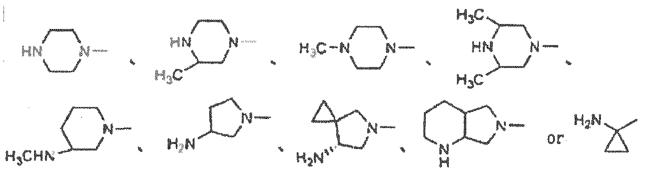 |
| R⁵ | H, F, Cl or OCH₃ |
| R⁶ | H or C1-C6 Alkyl |
| R⁷ | C1-C6 Alkyl |

A further embodiment of the present invention is:
The alkoxy carbonyl hemiacetal-type ester compound, wherein the ester compound corresponds to Z = C and m = 1 in the chemical formula (II) for the alkoxy carbonyl hemiacetal-type ester of the pyridone carboxylic acid antibacterial drug, wherein the ester compound is selected from the "hemiacetal-type ester derivative" of the chemical formula (II-1): 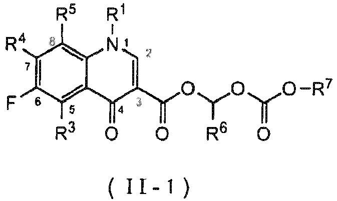 , which has the combination of each substituent R⁵, R³, R⁴, R⁵, R⁶, and R⁷, and is selected from:
   alkoxy carbonyl hemiacetal-type ester of Norfloxacin, wherein R¹ - ethyl, R³ = H, R⁴ = 1-piperazinyl, R⁵ = H, R⁶= H or C₁-C₆ alkyl, and R⁷ = C₁-C₆ alkyl;
   alkoxy carbonyl hemiacetal-type ester of Lomefloxacin, wherein R¹ = ethyl, R³ = H, R⁴ = 3-methyl-1-piperazinyl, R⁵= F, R⁶ = H or C₁-C₆ alkyl, and R⁷ =C₁-C₆ alkyl;
   alkoxy carbonyl hemiacetal-type ester of Fleroxacin, wherein R¹ = CH₂CH₂F, R³= H, R⁴ = 4-methyl-1-piperazinyl, R⁵= F, R⁶ = H or C₁-C₆ alkyl, and R⁷ = C₁-C₆ alkyl;
   alkoxy carbonyl hemiacetal-type ester of Ciprofloxacin, wherein R¹ = cyclopropyl, R³ = H, R⁴ = 1-piperazinyl, R⁵ = H, R⁶= H or C₁-C₆ alkyl, and R⁷ = C₁-C₆ alkyl;
   alkoxy carbonyl hemiacetal-type ester of Sparfloxacin, wherein R¹ = cyclopropyl, R³=NH₂, R⁴=3, 5- dimethyl- 1-piperazinyl, R⁵=F, R⁶ =H or C₁-C₆ alkyl, and R⁷ =C₁-C₆ alkyl;
   alkoxy carbonyl hemiacetal-type ester of Grepafloxacin which is R¹ = cyclopropyl, R³=CH₃, R⁴ =3- methyl-1-piperazinyl, R⁵=H, R⁶=H or C₁-C₆ alkyl, and R⁷ =C₁-C₆ alkyl;
   alkoxy carbonyl hemiacetal-type ester of Balofloxacin, wherein R¹ = cyclopropyl, R³=H, R⁴ =3-methylamino-1-piperidino, R⁵=OCH₃, R⁶=H or C₁-C₆ alkyl, and R⁷ =C₁-C₆ alkyl;
   alkoxy carbonyl hemiacetal-type ester of Temafloxacin, wherein R¹=2,4-difluorophenyl, R³=H, R⁴=3-methyl-1-piperaziny R⁵ H, R⁶=H or C₁-C₆ alkyl, and R⁷=C₁-C₆ alkyl; and
   alkoxy carbonyl hemiacetal-type ester of Sitafloxacin, wherein
      R¹= 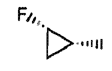
      ,R³=H,
      R⁴= 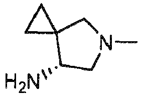
      , R⁵ =Cl, R⁶=H or C₁-C₆ alkyl, and R⁷ =C₁-C₆ alkyl.

These combinations of combination of each substituent R⁵, R³, R⁴, R⁵, R⁶, and R⁷ are collectively represented in table 8:

**Table 8**

| Hemiacetal type esters of NQ antibacterial drugs | | | | | | |
|---|---|---|---|---|---|---|
| 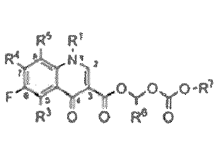 | | | | | | |
| | R¹ | R³ | R⁴ | R⁵ | R⁶ | R⁷ |
| Norfloxacin hemiacetal type ester | C₂H₅ | H | 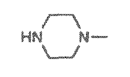 | H | H or C1-C6 alkyl | C1-C6 alkyl |
| Lomefloxacin hemiacetal type ester | C₂H₅ | H | 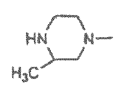 | F | | |
| Fleroxacin hemiacetal type ester | CH₂CH₂F | H | 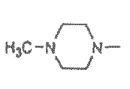 | F | ibid | ibid |
| Ciprofloxacin hemiacetal type ester | 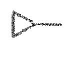 | H | 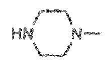 | H | ibid | ibid |
| Sparfloxacin hemiacetal type ester | 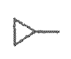 | NH₂ | 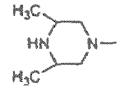 | F | ibid | ibid |
| Grepafloxacin hemiacetal type ester | 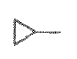 | CH₃ | 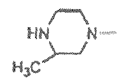 | H | ibid | ibid |
| Balofloxacin hemiacetal type ester | 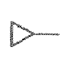 | H | 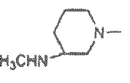 | OCH₃ | ibid | ibid |
| Temafloxacin hemiacetal type ester | 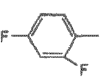 | H | 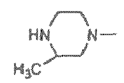 | H | ibid | ibid |
| Sitaflofloxacin hemiacetal type ester | 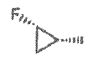 | H | 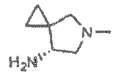 | Cl | ibid | ibid |

A further embodiment of the present invention is:
The alkoxy carbonyl hemiacetal-type ester compound, which has the combination of each substituent R¹¹, R³, R⁴, R⁶, and R⁷ in alkoxy carbonyl hemiacetal-type ester of the pyridone carboxylic acid antibacterial drug represented by the chemical formula (III) and is at least one alkoxy carbonyl hemiacetal-type ester of the pyridone carboxylic acid antibacterial drug selected from the followings:
   alkoxy carbonyl hemiacetal-type ester of Ofloxacin, wherein R¹¹= methyl, R³=H, R⁴=4-methyl-1-piperazinyl, R⁶=H or C₁-C₆ alkyl, and R⁷ =C₁-C₆ alkyl,
   alkoxy carbonyl hemiacetal-type ester of Levofloxacin, wherein R¹¹ 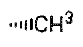
, R³=H, R⁴=4-methyl-1-piperazinyl, R⁶=H or C₁-C₆ alkyl, and R⁷ =C₁-C₆ alkyl, and
   alkoxy carbonyl hemiacetal-type ester of the Pazufloxacin, wherein R¹¹= 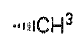
, R³=H, R⁴=1-amino-1-cyclopropyl, R⁶ H or C₁-C₆ alkyl, and R⁷=C₁-C₆ alkyl.

These combinations of each substituent R¹¹, R³, R⁴, R⁶, and R⁷ are collectively represented in the table 9:

**Table 9**

| Hemiacetal type esters of oxazine ring-containing NQ antibacterial drugs | | | | | | |
|---|---|---|---|---|---|---|
| 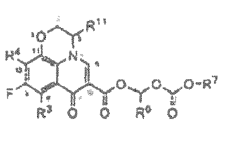 | | | | | | |
| | R¹¹ | R³ | R⁴ | R⁵ | R⁶ | R⁷ |
| Hemiacetal type ester of Ofloxacin | 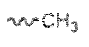 | H | 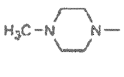 | H | H or C1-C6 alkyl | C1-C6 alkyl |
| Hemiacetal type ester of Levofloxacin | 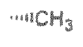 | H | 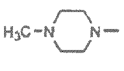 | H | ibid | ibid |
| Hemiacetal type ester of Pazufloxacin | 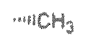 | H | 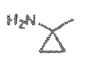 | H | ibid | ibid |

A further embodiment of the present invention is:
The alkoxy carbonyl hemiacetal-type ester compound, wherein the ester compound corresponds to Z = N and m = 0 in the chemical formula (II) for the alkoxy carbonyl hemiacetal-type ester of the pyridone carboxylic acid antibacterial drug, wherein the ester compound is selected from the hemiacetal-type ester derivative of the chemical formula (VII): 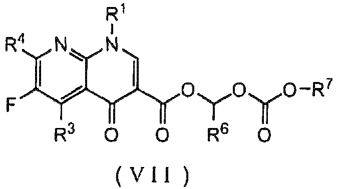 , wherein R¹ = ethyl, R³=H, R⁴=1-piperazinyl, R⁶=H or C₁-C₆ alkyl, and R⁷ =C₁-C₆ alkyl (that is; alkoxy carbonyl hemiacetal-type ester of Enoxacin), or R¹ =2,4-difluorophenyl, R³=H, R⁴ =3-amino-1-pyrrolidinyl, R⁶=H or C₁-C₆ alkyl, and R⁷ =C₁-C₆ alkyl (that is; alkoxy carbonyl hemiacetal-type ester of Tosufloxacin).

The combinations of the substituent in these chemical formula (VII) are summarized as A and B, and they are represented in table 10:

**Table 10**

| Substituents | R¹ | R³ | R⁴ | R⁶ | R⁷ |
|---|---|---|---|---|---|
| Combination A | C₂H₅ | H | 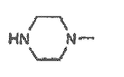 | H or C1-C6 alkyl | C1-C6 alkyl |
| Combination B | 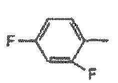 | H | 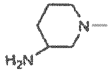 | ibid | ibid |

A further embodiment of the present invention is:
The alkoxy carbonyl hemiacetal-type ester compound, wherein the alkoxy carbonyl hemiacetal-type ester compound of the pyridone carboxylic acid antibacterial drug is at least one alkoxy carbonyl hemiacetal-type ester selected from: ethoxycarbonyl 1-ethyl hemiacetal-type ester of Levofloxacin represented by the chemical formula (V): 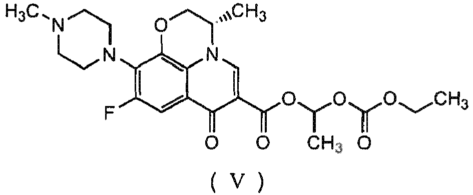 , isopropoxycarbonyl-1-ethyl hemiacetal-type ester of Levofloxacin represented by the chemical formula (VI): 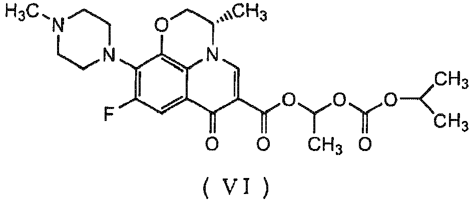 , ethoxycarbonyl 1-ethyl hemiacetal-type ester of Enoxacin represented by the chemical formula (VIII): 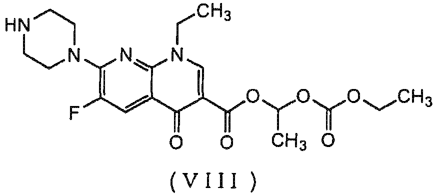 , isopropoxycarbonyl-1-ethyl hemiacetal-type ester of Enoxacin represented by the chemical formula (IX): 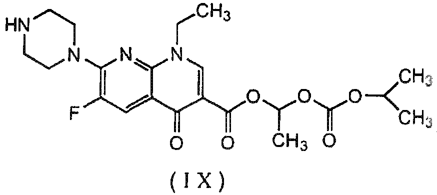 , ethoxycarbonyl 1-ethyl hemiacetal-type ester of Tosufloxacin represented by the chemical formula (X): 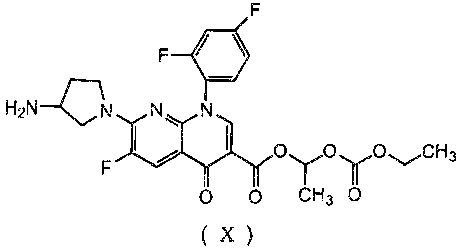 , and isopropoxycarbonyl-1-ethyl hemiacetal-type ester of Tosufloxacin represented by the chemical formula (XI):

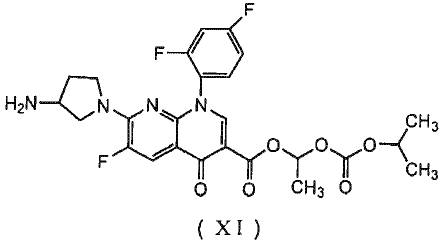

A further embodiment of the present invention is:
A method for producing an alkoxy carbonyl hemiacetal-type ester of the pyridone carboxylic acid antibacterial drug comprising reacting pyridone carboxylic acid represented by the chemical formula (I-1): 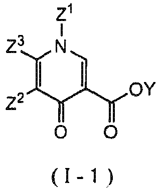 , wherein Z¹-Z³ are the same as above, and Y is H, alkali metal, alkaline earth metal or organic base, or its salt with alpha-halogenized dialkyl carbonate represented by the chemical formula (XII): 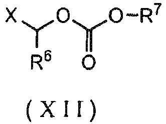 , wherein, R⁶ and R⁷ are the same as above, and X is halogen selected from Cl, Br, and I. to produce an alkoxy carbonyl hemiacetal-type ester of the pyridone carboxylic acid antibacterial drug represented by chemical formula (I): 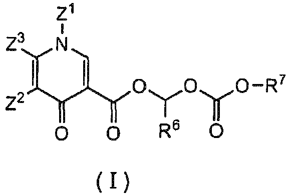 , wherein Z¹-Z³, R⁶, and R⁷ are the same as above.

A further embodiment of the present invention is:
The method for producing an alkoxy carbonyl hemiacetal-type ester, wherein the pyridone carboxylic acid or its salt is pyridone carboxylic acid represented by the chemical formula (II-A) or its salt: 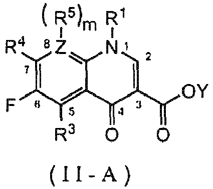
   , wherein m represent 1 or 0,
   R¹ is C₁-C₄ alkyl optionally substituted with halogen, C₃-C₆ cycloalkyl optionally substituted with halogen, or phenyl optionally substituted with halogen, R³ represents H, NH², or methyl,
   R⁴ represents: 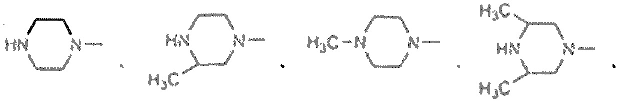 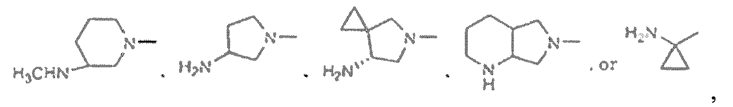
   R⁵ represents H, F, Cl, or OCH₃, or
   R⁵ and R¹ may join together with N of the 1st position and Z of the 8th position to form a nitrogen-containing heterocycle, or nitrogen- and oxygen-containing heterocycle optionally substituted (provided that; when Z = N, m = 0), Y is H, alkali metal, alkaline earth metal or organic base, and
   , the ester obtained is alkoxy carbonyl hemiacetal-type ester of the pyridone carboxylic acid antibacterial drug represented by the chemical formula (II): 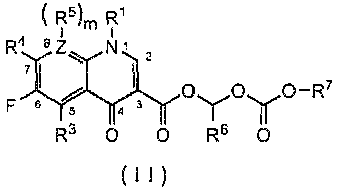 , wherein m, R¹, R³-R⁷, and Z are the same as above.

A further embodiment of the present invention is:
The method for producing the alkoxy carbonyl hemiacetal-type ester, wherein the pyridone carboxylic acid or its salt is pyridone carboxylic acid represented by the chemical formula (III-1) or the salt thereof: 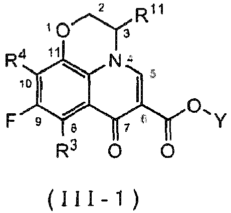
   , wherein R³ represents H, NH₂ or methyl, R⁴ represents: 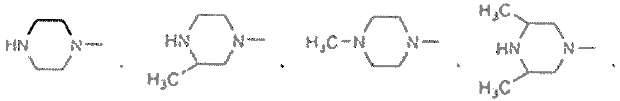 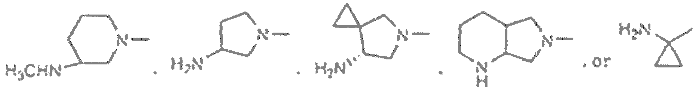
   , Y is H, alkali metal, alkaline earth metal or organic base, R¹¹ is 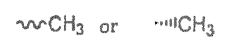
   , and the ester obtained is alkoxy carbonyl hemiacetal-type ester of the pyridone carboxylic acid antibacterial drug represented by the chemical formula: (III): 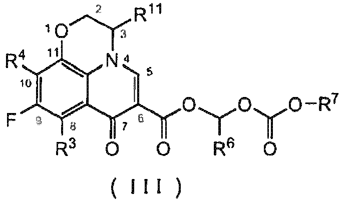 , wherein R³-R⁴, R⁶-R⁷, and R¹¹ are the same as above.

A further embodiment of the present invention is:
The method for producing the alkoxy carbonyl hemiacetal-type ester, wherein NaI is made to coexist together when X= Cl or Br in the alpha-halogenized dialkyl carbonate of the chemical formula (XII).

A further embodiment of the present invention is:
The method for producing the alkoxy carbonyl hemiacetal-type ester, wherein the method is carried out in the presence of at least one base selected from inorganic base or organic base.

A further embodiment of the present invention is
The method for producing the alkoxy carbonyl hemiacetal-type ester, wherein the pyridone carboxylic acid or its salt is represented by the chemical formula (II-A): 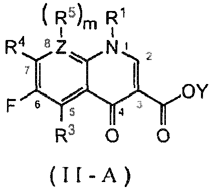 , wherein R⁵, R³, R⁴, R⁵, Z, m, and Y are the same as above, and further at least one of substituents R¹ and R³-R⁵ is a substituent comprising an active amino group, wherein the method is for producing an alkoxy carbonyl hemiacetal-type ester represented by the chemical formula (II): 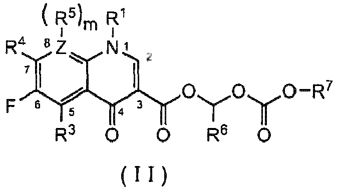 , wherein m, R⁵, R³-R⁷, and Z are the same as above,
, wherein the method is characterized by comprising the sequential steps of:
(i) protecting the active amino group by benzyloxycarbonyl,
(ii) reacting alpha-halogenized dialkyl carbonate represented by the chemical formula (XII) to obtain a hemiacetal-type ester derivative, and
(iii) removing the benzyloxycarbonyl protecting group by catalytic hydrogenation decomposition.

A further embodiment of the present invention is:
The method for producing the alkoxy carbonyl hemiacetal-type ester, wherein the active amino group is cyclic secondary amine.

A further embodiment of the present invention is:
The method for producing an alkoxy carbonyl hemiacetal-type ester, wherein the cyclic secondary amine is at least one cyclic secondary amine selected from the following chemical formula:

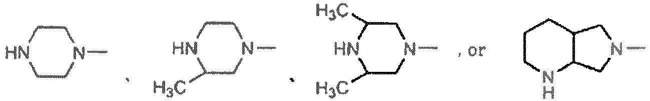

### EFFECT OF INVENTION

Because a fall of a blood concentration was hardly observed even if the simultaneous concomitant use of the alkoxy carbonyl hemiacetal-type ester prodrug of the pyridone carboxylic acid antibacterial drug of the present invention with a polyvalent metal-containing preparation such as antacid was carried out, it was confirmed that the absorption from intestinal tract is excellent while keeping the ester derivative structure, and the absorption inhibition by chelate formation is prevented.

Moreover, since the elevation of the concentration in blood of the pyridone carboxylic acid antibacterial drug was also prompt both in the in-situ experiment using a rat inversion intestinal tract and in the oral administration experiment, it was also confirmed that the prodrug of the present invention functions as a prodrug by being hydrolyzed within 1 hour promptly in a small intestine brush border membrane after absorption, and the pyridone carboxylic acid antibacterial drug as a drug substance is regenerated.

Furthermore, with regard to the alkoxy carbonyl hemiacetal-type ester derivative prodrug of the pyridone carboxylic acid antibacterial drug of the present invention, the antibacterial activity was found to decrease greatly in the alkoxy carbonyl hemiacetal-type ester derivative prodrug as compared to the drug substance (refer to Example 5, as stated later) as a result of the antibacterial activity measurement using Pseudomonas-aeruginosa ATCC27853, Escherichia coli ATCC25922 and NIHJC2 and Enterobacter ZTCC23355, and using an unesterified drug substance as control. Therefore, it was demonstrated that the ester prodrug of the present invention does not have an antibacterial activity against these kinds of bacteria before absorption from intestines. Accordingly, it was confirmed that the alkoxy carbonyl hemiacetal-type ester derivative prodrug of the present invention is a hybrid pyridone carboxylic acid antibacterial drug which minimizes influence on the normal bacterial flora in intestines, and thus, it reduces a risk of onset of pseudomembranous colitis until it is absorbed after oral administration.

Moreover, the esterification method of the present invention gives not only alkoxy carbonyl hemiacetal-type ester of the present invention in high yield, but it can selectively produce the aimed compound (alkoxy carbonyl hemiacetal-type ester derivative) in the esterification of the pyridone carboxylic acid antibacterial drug comprising especially an active amino group and a carboxyl group, by combining a protection of the active amino group by benzyloxycarbonyl and a deprotection by hydrogenation after esterification of the present invention. In this way, the esterification method of the present invention is an extremely excellent method.

Furthermore, as will be explained in full detail afterward, the alkoxy carbonyl hemiacetal part is by-produced when the pyridone carboxylic acid antibacterial drug as a drug substance is regenerated by hydrolysis after absorbed within inside of the intestinal tract, and the alkoxy carbonyl hemiacetal part decomposes non-enzymatic only to by-produce C₂-C₆ aldehyde, C₁-C₆ alcohol and CO₂, but does not by-produce a harmful C₁ aldehyde (formalin). This is another feature which shows the safety of the ester prodrug of the present invention.

### BRIEF DESCRIPTION OF DRAWINGS

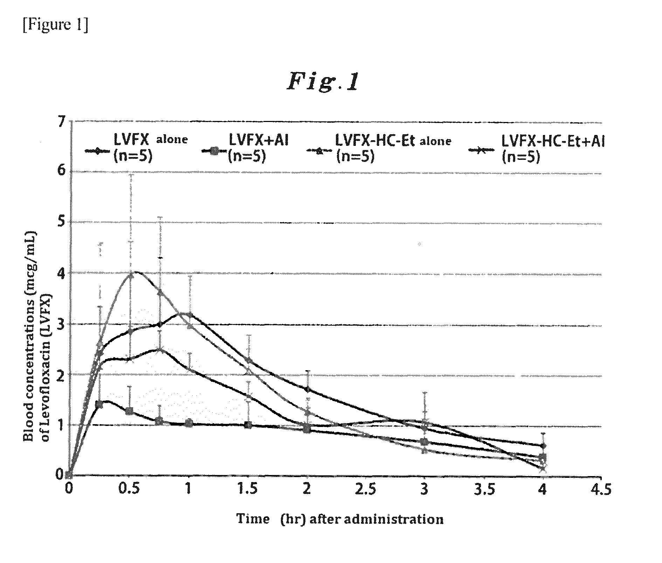

Fig. 1 represents the changes of the blood concentration of Levofloxacin at the time of independent oral administration of Levofloxacin ethoxycarbonyl-1-ethyl hemiacetal-type ester derivative (LVFX-EHE) and at the time of its combined oral administration with a metal-containing preparation to rats to which the present invention is applied.

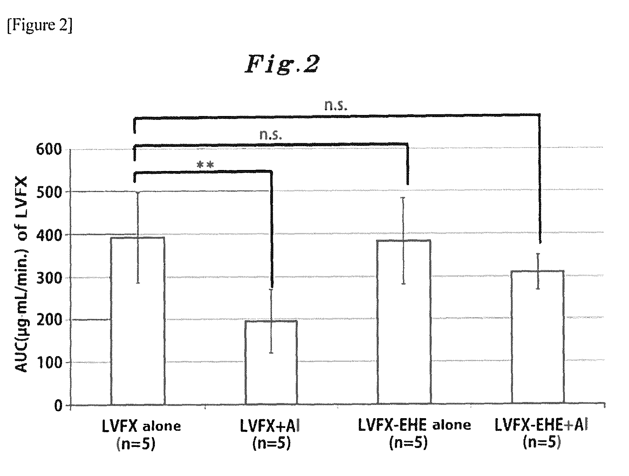

Fig. 2 represents the results of the computed area under the plasma drug concentration-time curve (AUC) in Fig. 1. Data are displayed as average ± S.D. (n= 5). n.s.: means "with no significant difference".

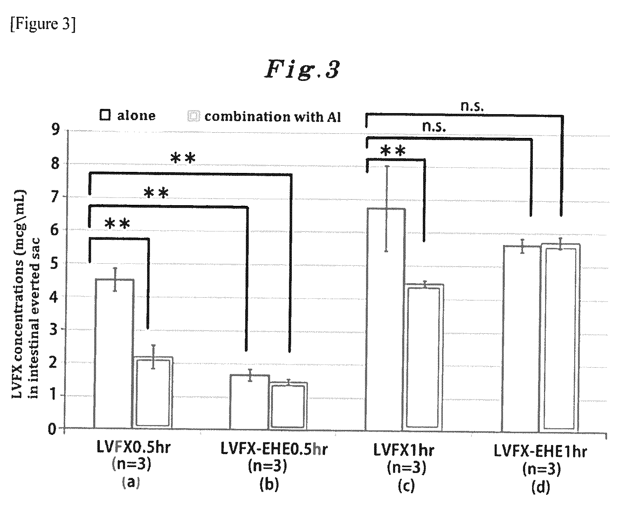

Fig. 3 represents the amounts of intestinal absorption of the alkoxy carbonyl hemiacetal-type ester prodrug (LVFX-EHE) in 0.5 hour and 1 hour by the rat intestinal everted sac study. Each bar represents the average ± S.D. (n= 3). **p < 0.01, n.s.: means "with no significant difference".

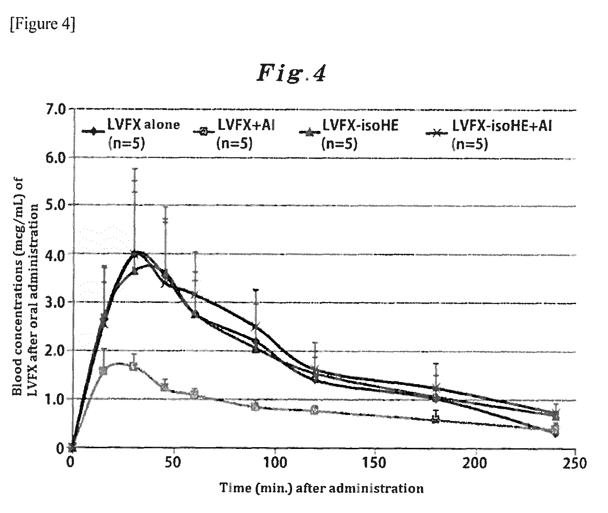

Fig. 4 represents the changes of the blood concentration of Levofloxacin at the time of independent oral administration of Levofloxacin isopropoxycarbonyl-1-ethyl hemiacetal-type ester derivative (LVFX-iPrHE) to rats and at the time of its combined oral administration with a metal-containing preparation to rats.

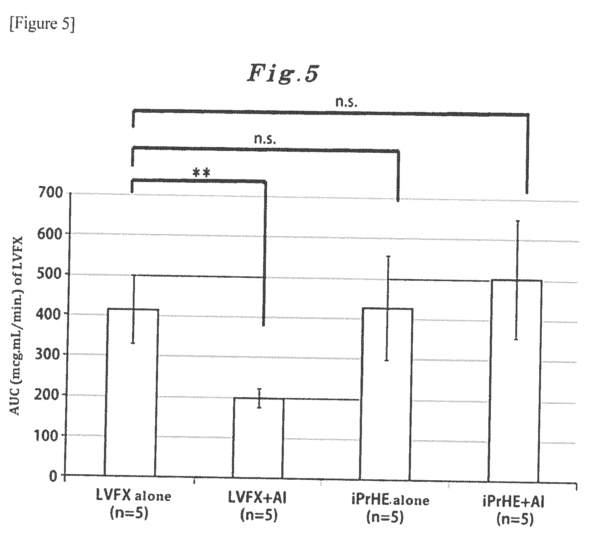

Fig. 5 represents the results of the computed-area under the plasma drug concentration-time curve (AUC) in Fig. 4. Data are displayed as average ± S.D. (n 5). *p < 0.05, **p < 0.01, n.s.: means "with no significant difference".

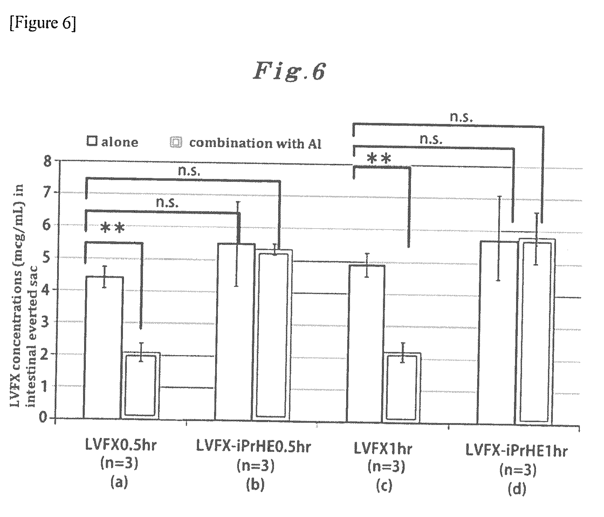

Fig. 6 represents the amounts of intestinal absorption of the alkoxy carbonyl hemiacetal-type ester prodrug (LVFX-iPrHE) at 0.5 hour and 1 hour by the rat intestinal everted sac study. Each bar represents the average ± S.D. (n = 3). *p < 0.05, **p <0.01, n.s.: means "with no significant difference".

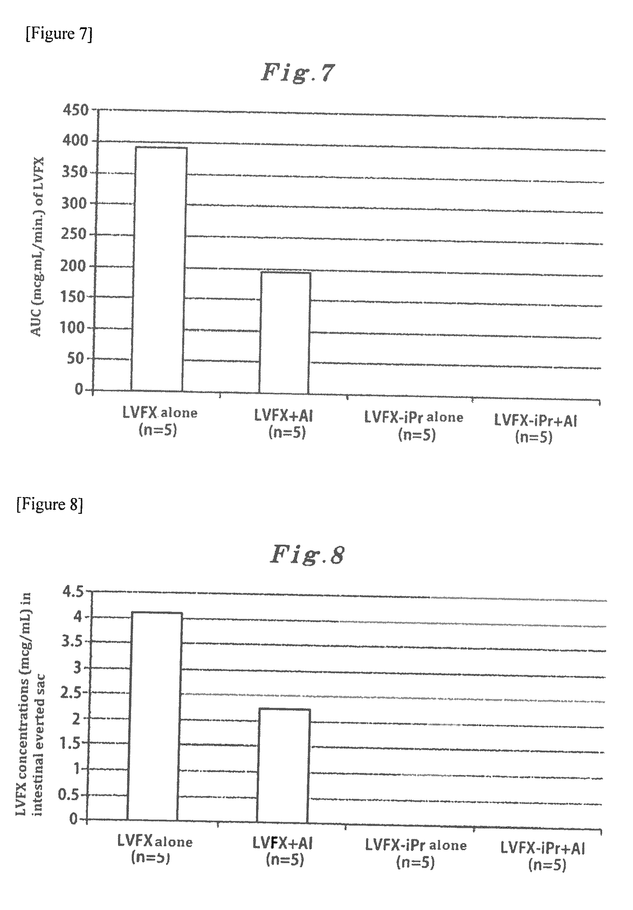

Fig. 7 represents the results of the computed-area under the plasma drug concentration-time curve (AUC) of Levofloxacin at the time of independent oral administration of Levofloxacin isopropyl ester (also expressed as LVFX-iPrE or LVFX-iPr) which is a simple ester derivative of Levofloxacin obtained in the reference synthesis example 2, and at the time of its combined oral administration with a metal-containing preparation Al(OH)₃ to male SD rats. The results are displayed as an average of ± S.D. (n = 5). * p < 0.05, **p < 0.01.
Fig. 8 represents Levofloxacin (LVFX) concentrations generated in the everted intestinal tract in the case of Levofloxacin isopropyl ester which is a simple ester derivative obtained in the reference synthesis example 2, and in the case of a concomitant use with a metal-containing preparation Al(OH)₃. Results are displayed as an average of ± S.D. (n= 5).*p<0.05,**p<0.01.

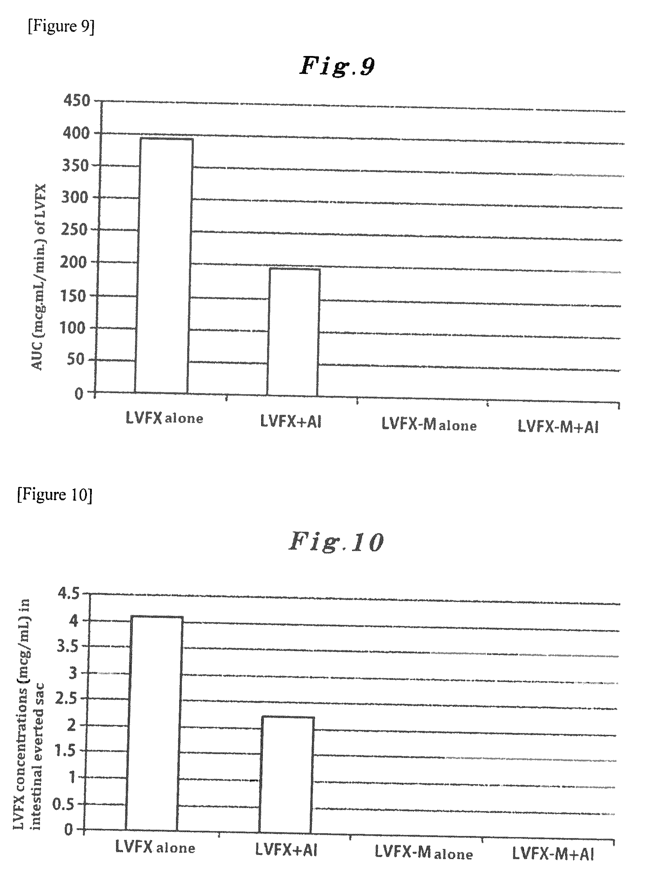

Fig. 9 represents the results of the computed-area under the plasma drug concentration-time curve (AUC) of Levofloxacin in the case of independent oral administration of Levofloxacin methyl ester (also expressed as LVFX-ME or LVFX-M) which is a simple ester derivative of Levofloxacin obtained in the reference synthesis example 1, and in the case of its combined oral administration with a metal-containing preparation Al(OH)₃ to rats.
Fig. 10 represents the Levofloxacin (LVFX) concentrations generated in the everted intestinal tract in the case of independent Levofloxacin methyl ester (LVFX-M) which is a simple ester derivative of Levofloxacin obtained in the reference synthesis example 1, and in the case of concomitant use of a metal-containing preparation Al(OH)₃.

### MODE FOR CARRYING OUT INVENTION

Hereinafter, the present invention is explained in detail.

One of the features of the present invention is that, by carrying out the alkoxy carbonyl hemiacetal-type esterification of the carboxyl group of the pyridone carboxylic acid antibacterial drug (quinolone type or new quinolone type carboxylic acid antibacterial drug), the resulting ester prodrug does not exhibit an antibacterial action during stay in intestines, therefore side effects such as pseudomembranous colitis are prevented because the ester prodrug does not kill good Enterobacterium and then, after being absorbed into the intestinal tract, the ester prodrug is hydrolyzed to regenerate the drug substance which exerts the antibacterial action of the pyridone carboxylic acid antibacterial drug.

Furthermore, it is also an excellent feature representing the safety aspect of the ester prodrug of the present invention that the alkoxy carbonyl hemiacetal part represented by the chemical formula: 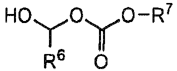 , wherein R⁶ and R⁷ are the same as above, decomposes non-enzymatically within inside of the intestinal tract, and does not produce a harmful C₁ aldehyde, but produces only C₂-C₆ aldehyde, C₁-C₆ alcohol and CO₂ as by-products following the reaction formula (1): 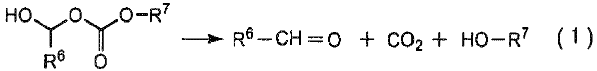 , wherein the above alkoxy carbonyl hemiacetal part is produced as a by-product when the pyridone carboxylic acid antibacterial drug as a drug substance is regenerated by hydrolysis after absorbed within the inside of the intestinal tract.

A further feature of the present invention by the esterification is prevention of insolubilization of the pyridone carboxylic acid antibacterial drug caused by the chelate formation at the time of concomitant use of the pyridone carboxylic acid antibacterial drug and a polyvalent metal-containing medicine (for example, polyvalent metal-containing antacids, iron preparations, etc.). Accordingly, the alkoxy carbonyl hemiacetal-type ester prodrug of the pyridone carboxylic acid antibacterial drug of the present invention can be administered substantially simultaneously with the polyvalent metal-containing medicine.

The pyridone carboxylic acid antibacterial drug as a drug substance of the prodrug of the present invention is represented by the chemical formula (I'): 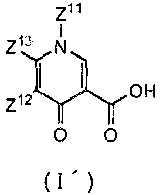 , wherein Z¹¹-Z¹³ are the same as above. Specific examples thereof include Nalidixic acid: chemical formula (1), and Pipemidic acid: chemical formula (2):

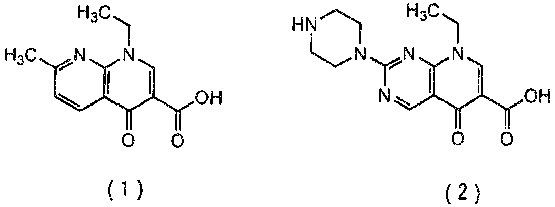

The pyridone carboxylic acid antibacterial drug is a general term comprising a quinolone carboxylic acid antibacterial drug (optionally called as "quinolone antibacterial drug"), and a new quinolone carboxylic acid antibacterial drug (optionally called as "new quinolone antibacterial drug").

The new quinolone antibacterial drug as a drug substance of the prodrug of the present invention is not limited specifically, but as typical examples, mentioned are fluorine substituted derivatives at 6th position of the quinolone ring represented by the chemical formula (A): 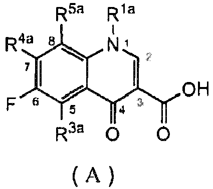 , wherein, R^{1a}, R^{3a}, R^{4a}, and R^{5a} are the same as above. Among them, Norfloxacin, Fleroxacin, Ciprofloxacin, Sparfloxacin, Grepafloxacin, Balofloxacin, Temafloxacin, Sitafloxacin, and Moxifloxacin are mentioned especially preferable.

On the other hand, oxazine ring-containing new quinolone antibacterial drugs represented by the chemical formula (B): 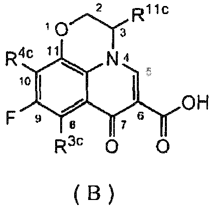 , wherein R³C, R^{4C} and R^{11C} are the same as above, are also typically mentioned as drug substances of the prodrug of the present invention, wherein the chemical formula (B) is of the type in which R^{1a} at the 1st position and R^{5a} at the 3rd position join together with an N atom and a C atom of the quinolone ring in the above-mentioned chemical formula (A) to form an oxazine ring. Among them, Ofloxacin, Levofloxacin and Pazufloxacin are selected preferably. Especially, Levofloxacin is mentioned preferably.

Moreover, naphthyridine type new quinolone antibacterial drugs represented by the chemical formula (C): 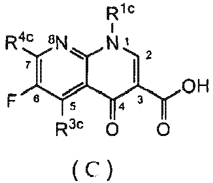 , wherein, R^{1C}, R^{3C} and R^{4C} are the same as above, are also typical new quinolones of the drug substances for the prodrug of the present invention, and, specifically, Enoxacin, Tosufloxacin, etc. are preferable.

The prodrugs of the present invention are alkoxy carbonyl hemiacetal-type ester derivatives which use the pyridone carboxylic acid antibacterial drugs (quinolone carboxylic acid antibacterial drug and new quinolone carboxylic acid antibacterial drug) as drug substances. The chemical formulas thereof are as follows: (i) the alkoxy carbonyl hemiacetal-type ester prodrug of the pyridone carboxylic acid antibacterial drug represented by the chemical formula (I): 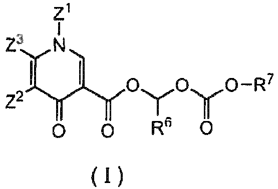 , wherein, Z¹-Z³, R⁶ and R⁷ are the same as above, and especially, (ii) the alkoxy carbonyl hemiacetal-type ester prodrug of the new quinolone antibacterial drug represented by the chemical formula (II): 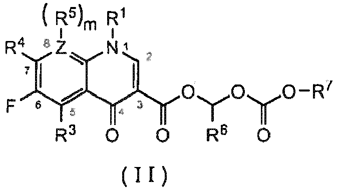 , wherein, m, R¹, and R³-R⁷ are the same as above.

Among these substituents, R⁶ is H or C₁-C₆ alkyl, and R⁷ is C₁-C₆alkyl. Here, the C₁-C₆ alkyl is selected from methyl, ethyl, 1-propyl, iso-propyl, 1-butyl, sec-butyl, 1-pentyl, 2-pentyl, 3-pentyl, 1-hexyl, 2-hexyl, 3-hexyl, or 4-hexyl.

Among these prodrugs, preferable is the alkoxy carbonyl hemiacetal-type ester prodrug of the oxazine ring-containing new quinolone, wherein the ester prodrug is represented by the chemical formula (III): 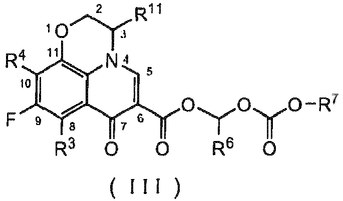 , wherein, R³, R⁴, R⁶, R⁷ and R¹¹ are the same as above, wherein the chemical formula (III) is formed by joining substituents R¹ and R⁵ together to form an oxazine ring in the chemical formula (II).

More specifically, the preferable alkoxy carbonyl hemiacetal-type ester of the present invention is an alkoxy carbonyl alkyl hemiacetal-type ester of Levofloxacin represented by the chemical formula (IV): 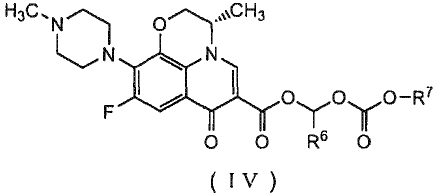 , wherein, R⁶ and R⁷ are the same as above.

Furthermore, most preferable are the ethoxycarbonyl 1-ethyl hemiacetal-type ester prodrug of Levofloxacin represented by the chemical formula (V): 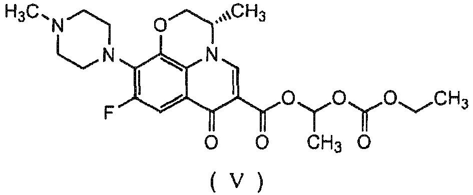 , and the isopropoxycarbonyl 1-ethyl hemiacetal-type ester prodrug of Levofloxacin represented by the chemical formula (VI):

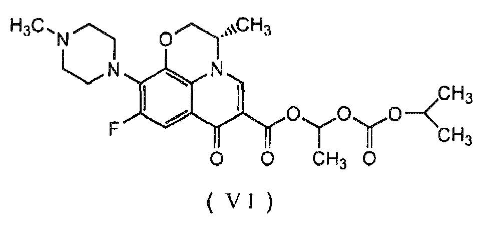

As preferable embodiments of prodrugs of the naphthyridine ring-containing new quinolone antibacterial drugs which correspond to Z = N in the chemical formula (II), illustrative examples include the ethoxycarbonyl 1-ethyl hemiacetal-type ester prodrug of Enoxacin represented by the chemical formula (VIII): 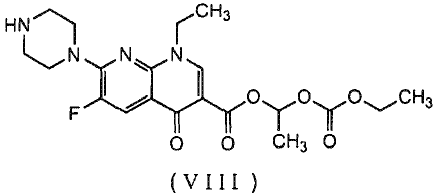 , and the isopropoxycarbonyl 1-ethyl hemiacetal-type ester prodrug of Enoxacin represented by the chemical formula (IX):

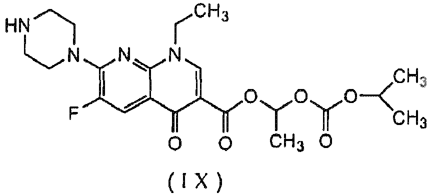

As preferable embodiments of prodrugs of naphthyridine-type new quinolone antibacterial drugs, illustrative examples include the ethoxycarbonyl 1-ethyl hemiacetal-type ester prodrug of Tosufloxacin represented by the chemical formula (X): 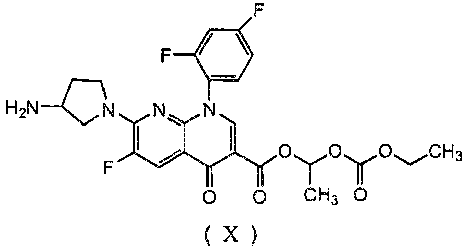 , and the isopropoxycarbonyl 1-ethyl hemiacetal ester type prodrug of Tosufloxacin represented by the chemical formula (XI):

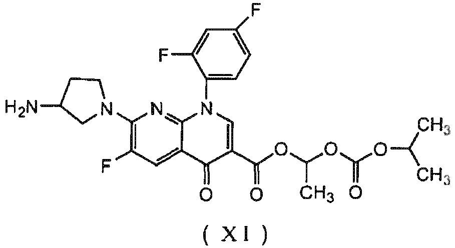

Next, a method is explained for producing the alkoxy carbonyl hemiacetal-type ester derivative of the pyridone carboxylic acid antibacterial drug of the present invention.

The method for producing the ester derivative comprises a step of reacting the carboxyl at 3-position of the new quinolones represented by the chemical formula (A-1) or the carboxyl of 6-position of the oxazine ring-containing new quinolones represented by the chemical formula (B-1) with alpha-halo dialkyl carbonate represented by the chemical formula (XII): 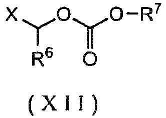 , wherein, R⁶ and R⁷ are the same as above, and X is halogen selected from Cl, Br, and I. according to the reaction scheme (2): 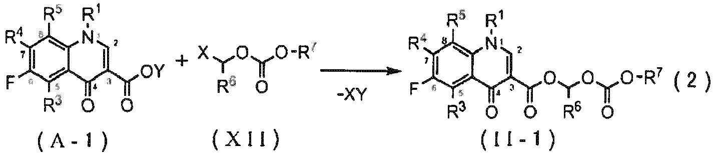 , wherein R¹ and R³-R⁷ are the same as above, Y is H, an alkali metal, an alkaline earth metal, or an organic base, and X represents halogen selected from Cl, Br and I, provided that, when X is Cl or Br, NaI may coexist, or more preferably, according to the reaction scheme (3): 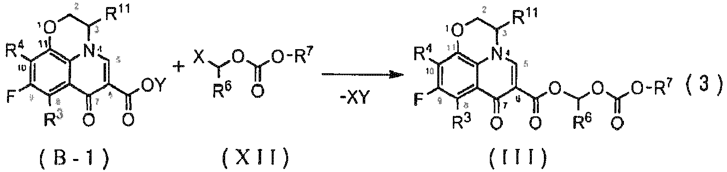 , wherein R³, R⁴, R⁶, R⁷, R¹¹, Y, and X are the same as above, and then, as a result of the above reactions, the alkoxy carbonyl hemiacetal-type ester derivative (II-1) or (III) is obtained.

Alpha-chloro dialkyl carbonate, alpha-bromo dialkyl carbonate, or alpha-iodo alkyl carbonate is used as alpha-halo dialkyl carbonate, and preferably alpha-chloro or alpha-bromo dialkyl carbonate is used. Substituent R⁶ of alpha-halo dialkyl carbonate (XII) is H or C₁-C₆ alkyl, and R⁷ is C₁-C₆ alkyl. Here, C₁-C₆ alkyl is selected from methyl, ethyl, 1-propyl, 2-propyl, 1-butyl, 2-butyl, 1-pentyl, 2-pentyl, 3-pentyl, 1-hexyl, 2-hexyl, 3-hexyl and 4-hexyl. Alpha-chloro dimethyl carbonate, alpha-chloro diethyl carbonate, alpha-chloro ethyl methyl carbonate, alpha-chloro ethyl n-propyl carbonate, alpha-di-n-propyl carbonate, and alpha-bromo forms thereof, etc. are mentioned as non-limiting examples,.

The amount of alpha-halo dialkyl carbonate used is influenced by reaction conditions, and it is one or more equivalents, generally 1 to 10 equivalents, preferably 1.5 to 8 equivalents, more preferably 2-7 equivalents to carboxyl.

Y in the chemical formula (A-1) and (B-1) which are the pyridone carboxylic acid part to be esterified is H (free carboxylic acid), an inorganic base such as alkali metal or alkaline earth metal, or an organic base such as amine (carboxylate). The alkali metal is selected from Li, Na, K, Rb, and Cs, but usually Na or K is used. The alkaline-earth metal is selected from Ca, Mg, Sr and Ba, but usually Ca or Mg is used. As the inorganic base, sodium carbonate, potassium carbonate, etc. are mentioned, and triethylamine, triisopropylamine, tri-n-butylamine, dimethylaminopyridine, or pyridine is mentioned as an organic base, for example. When Y is an inorganic or organic base except for free carboxylic acid in which Y is H, esterification can be carried out after making a salt form in-situ.

The esterification reaction can be carried out with or without a catalyst. However, in order to accelerate the esterification reaction, such a catalyst can be used as quaternary ammonium salt (for example, tetrabutylammonium bromide), alkali metal bromide or iodide (for example, NaBr or NaI), or cyclic ether. The amount of the catalyst used per 1 equivalent of alpha-halo dialkyl carbonate (XII) is 0.005-1.0 equivalents, preferably 0.01-0.6 equivalents, more preferably 0.1-0.4 equivalents.

The esterification reaction is carried out, preferably in organic reaction mediums, for example, N,N-dimethylformamide (DMF), N,N-dimethylacetamide , dimethyl sulfoxide, acetonitrile, toluene, xylene, dioxane, tetrahydrofuran, chloroform, methylene chloride, ethylene chloride, benzene, ethyl acetate, etc., or in the mixtures thereof at temperatures of 0 deg C - 100 deg C, preferably 10 deg C - 80 deg C, for example, 70 deg C.

In the esterification reaction, when the pyridone carboxylic acid antibacterial drug to be esterified contains an active amino group, the esterification after protecting the active amino group is desirable. The reason is to give priority to the esterification of the carboxyl group by suppressing the competitive reaction between the carboxyl group to be esterified and the active amino group. Conventionally, the Boc has been used as a protecting group of the active amino group. That is, the esterification of the active amino group is carried out after the active amino group is protected by the Boc group, and the Boc group is eliminated by hydrolysis after the esterification reaction. However, since the hemiacetal-type ester derivative of the present invention is very vulnerable to hydrolysis, the ester is also hydrolyzed in the hydrolysis process of the Boc group. Therefore, the synthesis of the aimed ester was not successful till now. According to the method of the present invention, the hemiacetal-type ester derivative of the carboxylic acid can be obtained in high yield by carrying out the hemiacetal-type esterification of the carboxylic acid after protecting the active amino group by a benzyloxycarbonyl group (Z group) and successively carrying out elimination of the Z group by a catalytic reduction method.

The reaction scheme is illustrated below using Enoxacin (optionally called "ENOX") which is one of the new quinolones having the active amino group:

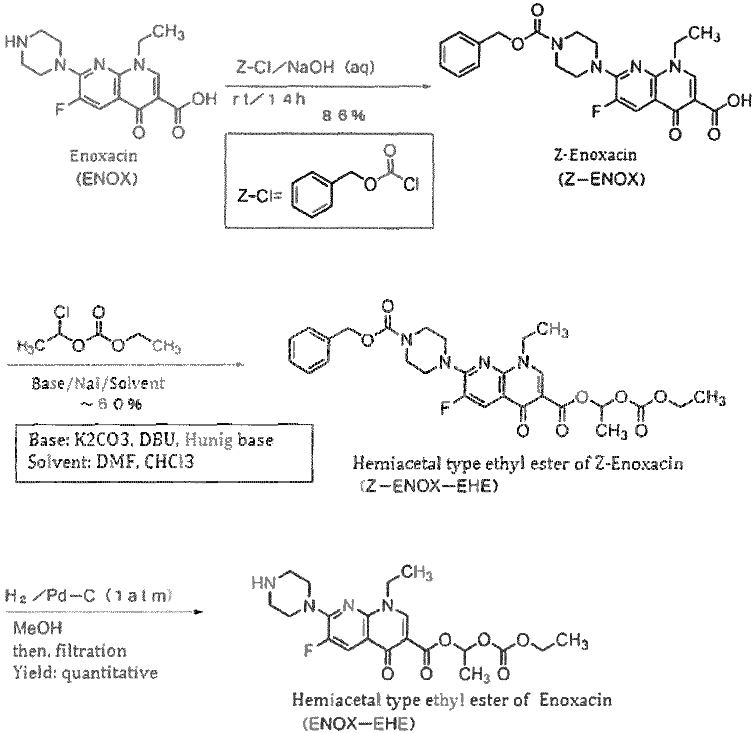

That is, Z-Cl and 2M-NaOH are added by turns to a dioxane solution of Enoxacin carefully so that the temperature may not exceed 10 deg C, and stirred at room temperature for 14 hours. Z-Enoxacin can be obtained with high yield (86%) by the steps of pH adjustment to weak acidic, filtering of a precipitate, washing by water, and drying. Subsequently, 1-chloroethylethyl carbonate and sodium iodide are reacted with Z-Enoxacin in the presence of a base (K₂CO₃, DBU, Hunig base, etc.), resulting in the production of Z-Enoxacin ethoxycarbonyl-1-ethyl hemiacetal-type ester in moderate chemical yield (around 60%) after purification by column chromatography. Successively, elimination of Z group is carried out by catalytic hydrogenolysis in methanol using a Pd-C catalyst in atmospheric pressure, and then, Enoxacin ethoxycarbonyl-1-ethyl hemiacetal-type ester can be obtained in high yield (84%).

The active amino group as used herein refers to an amino group with high nucleophilic reaction property, for example, a secondary cyclic amino group is mentioned. Specifically, cyclohexylamino, cyclopentylamino, etc. are mentioned as the active amino group used with the pyridone carboxylic acid antibacterial drug as shown below:

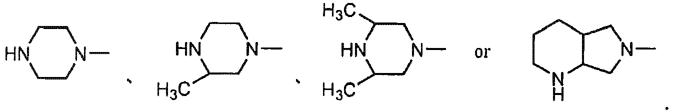

After completion of the esterification reaction, the product is isolated and purified through after-treatment with a conventional method. For example, warming is stopped, water and ice (optimum amount) are added for quenching the reaction, an extracting solvent (for example, AcOEt) is added for extraction, and the organic layer is separated from the aqueous layer. The aqueous layer is extracted with a new extracting organic solvent several times, and after the combined organic layer is washed successively with water, 1% sodium thiosulfate solution and then saturated sodium chloride aqueous solution, the organic layer is dehydrated with MgSO₄, and the filtered solution is concentrated in vacuum. The obtained crude product is purified, for example, by silica gel column chromatography, and the aimed hemiacetal-type ester derivative of the pyridone carboxylic acid antibacterial drug is obtained in high purity.

When the esterification reaction was carried out after protecting the active amino group by a Z group, the aimed ester derivative can be obtained after removing the Z group by the catalytic hydrogenolysis of the purified ester derivative or the unpurified ester derivative.

Identification of the obtained aimed compound was carried out by ¹H-NMR (400 MHz, CDCl₃), ¹³C-NMR (100 MHz, CDCl₃), HR-MS, and melting-point measurement (in the case of a crystal).

Then, a pharmaceutical composition comprising the prodrug of the present invention and pharmaceutically acceptable excipients is explained as follows.

The pharmaceutical composition of the present invention can be formulated for administration by mixing with nontoxic and inactive pharmaceutically acceptable excipients, for example, diluents, fillers and carriers in solid state, semi-solid state, or liquid state. The examples of such preparations are in the forms of tablets, troches, capsules, pellets, suppositories, solutions, suspensions, emulsions, etc.

The prodrug of the present invention can be mixed with conventional excipients or mixtures thereof in the case of tablets, troches, capsules, and pellets. The followings are mentioned as examples of the excipients: bulking agents and fillers, such as starch, lactose, sucrose, glucose and mannitol; binders, such as carboxymethyl cellulose, alginate, gelatin, and polyvinylpyrrolidone; disintegrating agents, such as calcium carbonate and sodium bicarbonate; absorption enhancers, such as quaternary ammonium compounds; moistening agents, such as cetyl alcohol and glycerol monostearate; adsorbents, such as kaolin and bentonite; lubricants, such as talc, calcium stearate, magnesium stearate, and solid polyethylene glycol. Tablets, troches, capsules, pills, and granules can be coated by conventional coating materials (opacifiers, for example).

Solution preparations or emulsion preparations for oral administration may comprise conventional solvents, solubilizers, emulsifiers, etc. other than the prodrug of the present invention, for example, water, ethyl alcohol, benzyl benzoate, propylene glycol, cottonseed oil, peanut oil, corn oil, olive oil, fat ester, for example, glycerin, polyethylene glycol, or fatty acid ester of the sorbitan, or mixtures thereof.

The preparation may also contain about 0.1 to 99.5% by weight, preferably 0.5 to 95% by weight of the prodrug of the present invention, and further contain dyes, preservatives, sweetening agents, and excipients in conventionally acceptable amounts.

The prodrug of the present invention may be administered orally or enterally. It is known generally that the active ingredient can be advantageously medicated in the amount of about 0.1-500, preferably 1-100 mg/kg weight per day (as the amount of the pyridone carboxylic acid antibacterial drug after the ester prodrug is hydrolyzed) as one does or divided doses. The dose in the range of about 1-200 mg/kg-weight, especially 1-50 mg/kg-weight is desirable. However, it will be understood that the amount of the compound to be administered actually may be controlled from viewpoints of various related environmental factors, for example, form of preparation, selected route of administration, each patient's age, weight, response, and the level of symptom of the patient.

The composition of the present invention is preferable to be administered in an unit dosage form. "The unit dosage form" used in the description is the composition of the present invention comprising the amount of the pyridone carboxylic acid antibacterial drug which is suitable for medicating the object of human or lower animal in one time of the administration dose in accordance with the implementation of good medical care. The composition comprises from about 30 mg, preferably from about 50 mg, more preferably from about 100 mg, further preferably from about 200 mg, up to about 20,000 mg, preferably to about 7,000 mg, still more preferably to about 1,000 mg, still more preferably to about 500 mg of the pyridone carboxylic acid antibacterial drug.

A further embodiment of the present invention provides a kit which consists of the pharmaceutical composition and a polyvalent metal-containing medicine. For example, the polyvalent metal-containing medicine provides the pharmaceutical composition or kit, wherein the polyvalent metal-containing medicine is a polyvalent metal-containing antacid. Specifically, a kit combining a preparation containing aluminum hydroxide as a polyvalent metal antacid in the amount of 100 mg - 2000 mg, preferably 200 mg - 1000 mg with Levofloxacin as a typical drug of new quinolone antibacterial drugs in the amount of 100 mg can be provided.

A further embodiment of the present invention provides a pharmaceutical composition which can be administered orally and has a preventive action to the enteric-bacterium shift side effect.

### EXAMPLES

The present invention is explained specifically by the following examples. However, the present invention is not intended to be limited thereto.

### Example 1.

### Synthesis of Levofloxacin ethoxycarbonyl-1-ethyl hemiacetal-type ester (optionally abbreviated as "LVFX-EHE")

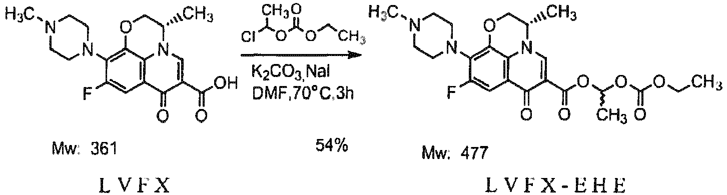

To a DMF (80 mL) solution of Levofloxacin (3.62 g, 10 mmol) in a 200-mL eggplant-shaped flask (a stirring bar contained) were added NaI (3.6 g, 24 mmol) and K₂CO₃ (3.3 g, 24 mmol), and the mixture was stirred. Subsequently, 1-chloroethyl-ethyl carbonate (8.6 mL, 64 mmol) was added thereto, and the mixture was stirred at 70 deg. C for 3 hours under argon gas flow. Warming was stopped and water (about 70 mL) and ice (optimum amount) were added for quenching the reaction. AcOEt (70 mL) was added for extraction, and the oil layer and the aqueous layer were separated. The extraction operation was carried out for the aqueous layer twice (50 mL x 2 times) by new AcOEt; the combined organic layer was dehydrated by MgSO₄ after one wash with water, one wash with a 1% sodium thiosulfate solution, and two wash with a saturated saline solution; and the filtered solution was concentrated in vacuum. A crude product (4.0 g) was obtained, and purified by silica gel column chromatography (an eluent is CHCl₃: MeOH=2: 1), and the object product was obtained in a yield of 2.58 g (54%). The results of analyses of this product are listed below, and the product was confirmed to be the aimed Levofloxacin ethoxycarbonyl-1-ethyl hemiacetal-type ester (LVFX-EHE).

m.p.: 167 to 173 deg. C.
¹H-NMR(400 MHz, CDCl₃);1.31 (t, 3H, J= 7.0 Hz, CH₃), 1.56 (d, 3H, J= 7.0 Hz, CH₃), 1.66 (d, 3H, J= 6.0 Hz, CH₃), 2.36 (s, 3H, CH₃), 2.56 (m, 4H, CH₂x2), 3.36 (m, 4H, CH₂x2), 4.23 (q, 2H, J= 7.0 Hz, CH₂), 4.31 (m, 1H), 4.36 (s, 2H, CH₂x2), 7.00 (q, 1H, J= 5.5 Hz, CH), 7.60*, 7.65* (d, 1H, JP 5.5 Hz), 8.26*, 8.27*(s, 1H, CH);
Note: Signal * indicates a separated diastereoisomer peak.
¹³C-NMR, (100 MHz, CDCl₃); 1418.3, 19.7, 46.4-50.5*, 50.6*, 54.7-55.7*, 55.8*, 64.3, 68.0, 91.5*, 91.6*, 105.1,107.9,108.0,123.0*, 123.1*, 123.5,131.8*, 131.9*, 139.6*, 139.7*, 145.3,145.8,153.0*, 153.1*, 154.5*, 155.0*, 156.9*, 157.1*, 162.4,163.2,172.3*, 172.5*. Note: Signal * indicates a separated diastereoisomer peak.
HR-MS; Calcd. for C₂₃H₂₈FN₃O₇: 477.1911, Found: 477.1909.

### Example 2

### Synthesis of Levofloxacin isopropoxycarbonyl 1-ethyl hemiacetal-type ester (optionally abbreviated as "LVFX-isoHE" or "LVFX-iPrHE")

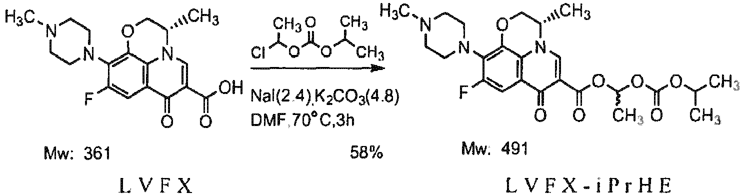

In a 300-mL eggplant-shaped flask, a DMF (110 mL) solution of Levofloxacin (10.0 g, 27.6 mmol) is prepared, and NaI (2.4 g, 66.24 mmol) and K₂CO₃ (4.8 g, 132.5 mmol) were added thereto, and the mixture was stirred. Subsequently, 1-chloroethyl-isopropyl-carbonate (27.0 mL, 176.64 mmol) was measured by a measuring cylinder, and was added to the flask with a pipette. After a Dimroth reflux condenser was attached, the reaction mixture was warmed at 70 deg. C and stirring was continued for 3 h. Then, water (about 100 mL) was slowly added to the mixture, and the reaction was quenched. After a proper quantity of ice was added also, the mixture solution was moved to a 300-mL separating funnel. AcOEt (70 mL) was added to the funnel, and it was shaken well, then, the AcOEt layer was separated. The aqueous layer was extracted twice by new AcOEt (50 mL), and the organic layers were combined. The combined organic layer was dehydrated with MgSO₄ after one wash with a 1% sodium thiosulfate solution, and one wash with saturated saline solution; the solution was filtered and the filtered solution was concentrated in vacuum. The residue (10.1g) was obtained.

Purification by column chromatography (developing solvent CHCl₃:MeOH=2:1) of the residue afforded the product (7.8 g; yield: 58%). The results of analyses of the product are described below, and the product was confirmed to be a Levofloxacin isopropoxycarbonyl 1-ethyl hemiacetal-type ester derivative (LVFX-isoPrHE).

m.p.: 132 to 133 deg. C.
¹H-NMR(400 MHz, CDCl₃);1.30*, 1.32* (d, 6H, J= 6.2 Hz), 1.53*, 1.54* (d, 3H, J=4.0 Hz), 1.65 (d, 3H, J= 5.5 Hz), 2.36 (s, 3H), 2.54 (m, 4H), 3.35 (m, 4H), 4.30-4.41 (m, 3H), 4.91 (sept, 1H, J= 6.2 Hz), 7.00*, 7.01 (q, 1H, J= 5.5 Hz), 7.56*, 7.59* (d, 1H, J= 13.0 Hz), 8.24*, 8.25* (s, 1H). Note: Signal * indicates a separated diastereoisomer peak.
¹³C-NMR, (100 MHz, CDCl₃); 50.5*, 50.6*, 54.8, 55.7, 64.3*, 64.4*, 68.1, 91.6*, 91.7*, 105.4*, 105.5*, 105.6*, 105.7*, 108.2*, 108.4*, 123.28*, 123.30*, 123.36*, 123.38*, 123.53,123.56*, 123.58*, 131.80*, 131.84*, 131.95*, 131.98*, 139.65*, 139.71*, 145.46*, 145.72*, 153.06*, 153.17*, 154.61*, 154.69*, 157.08*, 157.16*, 162.72*, 163.27*, 172.46*, 172.48*, 172.61*, 172.64*. Note: Signal * indicates a separated diastereoisomer peak.
HR-MS: Calcd. for C₂₄ H₃₀ FN₃ O₇: 491.2068; Found: 491.2069.

### Example 3

### Synthesis of Enoxacin ethylcarbonyl 1-ethyl hemiacetal-type ester (optionally abbreviated as "ENOX-EHE")

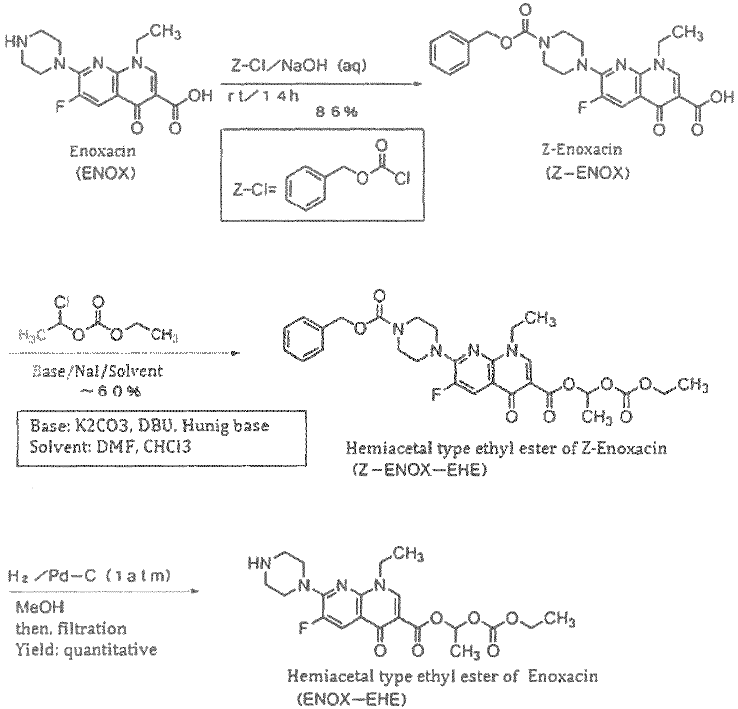

Z-Cl (1.85 mL, 13 mmol) and 2M NaOH (16 mL) were added alternately in three portions to Enoxacin (3.47 g, 10 mmol) while taking care for the reaction temperature not to exceed 10 deg. C, and the mixture was stirred at room temperature for 1.5 hours. The pH of the mixture was modulated into weakly acidic and the resulting precipitate was filtered and washed with water and dried. Z-Enoxacin (3.9 g, 86%) was obtained. Subsequently, Z-Enoxacin (0.91 g, 2 mmol) and 1-chloroethylethyl carbonate (0.8 mL, 6 mmol) were reacted with n-Bu₄NI (1.48 g, 4 mmol) in the presence of a base (DBU: 0.9 mL, 6 mmol) to afford Z-Enoxacin ethoxycarbonyl-1-ethyl hemiacetal-type ester in 60% yield after column chromatography purification. Furthermore, this Z-product was subjected to the catalytic hydrogenolysis in methanol using Pd-C catalyst in atmospheric pressure in order to eliminate the Z group, then, the aimed Enoxacin ethoxycarbonyl-1-ethyl hemiacetal-type ester was obtained in 84% yield.

1-Ethyl-6-tluoro-4-oxo-7-piperazine-1-yl-1,4-dihydro-[1,8]naphthyridine-3-carboxy acid 1-ethoxycarbonyloxy ethyl ester
¹H-NMR(400 MHz, CD₃OD):1.27 (t, 3H, J=7.3 Hz), 1.46 (t, 3H, J= 7.4 Hz), 1.60 (d, 3H, J= 5.5 Hz), 3.35 (m, 4H), and 4.03 (m, 4H), 4.18 (q, 2H, J= 7.3 Hz), 4.45 (q, 2H, J= 7.4 Hz), 6.92 (q, 1H, J= 5.5 Hz), 8.13 (d, 1H, J= 13.2 Hz), 8.76 (s, 1H).
Anal. Calcd. for C₂₀H₂₅N₄O₆F. C, 55.04; H, 5.77; N, 12.84; F, 4.32. Found; C, 55.00; H, 5.80; N, 12.81;F,4.32.

### Reference synthesis example 1

### Synthesis of Levofloxacin methyl ester (optionally abbreviated as "LVFX-M".)

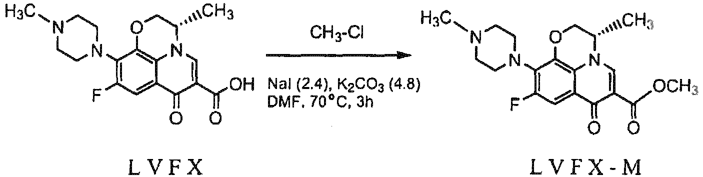

Levofloxacin methyl ester (LVFX-M) was obtained following the esterification reaction carried out according to Example 1 using methyl chloride in place of 1-chloroethyl isopropyl carbonate. Results of analyses of the obtained methyl ester are represented below:
(S)-Methyl-9-fluoro-3-methyl-10-(4-methylpiperazine-1-yl)-7-oxo-3,7-dihydro-2H-[1,4] oxazolino [2,3,4-ij] quinolin-6-carboxylate : (LVFX-M)
Chemical formula: C₁₉H₂₂FN₃O₄;
Theoretical value: 375.1594;
Molecular weight: 375.3941;
Elemental analysis: C, 60.79; H, 5.91; F, 5.06; N, 11.19; O, 17.05.

### Reference synthesis example 2

### Synthesis of Levofloxacin simple ester derivative: Isopropyl ester derivative (Optionally abbreviated as "LVFX-iso" or "LVFX-iPrE".)

According to the reaction scheme below, LVFX-iPrE was obtained by reacting an acid chloride form of Levofloxacin with isopropyl alcohol in the presence of a base.

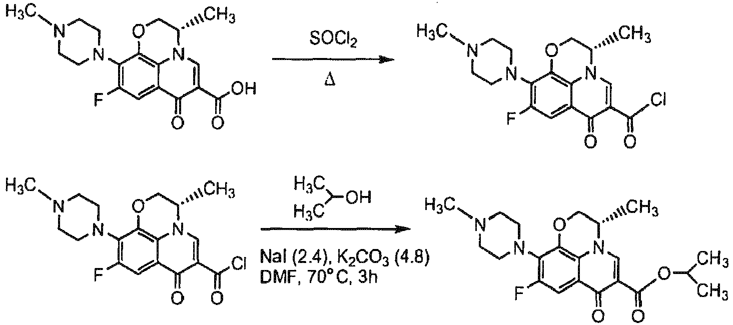

Results of analyses of the obtained product are represented below:.
[LVFX-isoPrE]
(S)-Isopropyl-9-fluoro-3-methyl-10-(4-methylpiperazine-1-yl)-7-oxo-3,7-dihydro-2H-[1, 4] oxazolino [2,3,4-ij] quinolin-6-carboxylate;
Chemical formula: C₂₁H₂₆FN₃O₄
Theoretical value: 403.1904;
Molecular weight: 403.445.

Then, the test methods used for the functional assay of the present invention are explained.

### Experimental materials and assay procedures are as follows.

### (1) Experimental materials

Levofloxacin (optionally abbreviated as "LVFX") used as a substrate drug and Ciprofloxacin (optionally abbreviated as "CPFX") used as an internal standard substance were purchased from Tokyo Chemical Industry Co., Ltd. (Tokyo).

As a polyvalent metal-containing preparation, aluminum hydroxide gel (Alumigel (registered trademark): Al(OH)₃; purchased from Yoshida Pharmaceutical Co., Ltd. (Tokyo)) was used for in-vivo testing (for administration to mice). On the other hand, water-soluble aluminum chloride (AlCl₃) was purchased from Yoshida Pharmaceutical Co., Ltd. (Tokyo) and used for in-vitro testing as a model of the polyvalent metal-containing preparation. Because aluminum hydroxide gel is not soluble in water, aluminum chloride was used as a sauce of Al³⁺ ion to verify chelation. All other reagents used were of the special chemical grade or the reagent for HPLC.

### (2) Experimental animals

The Sprague-Dawley (SD) type male rats (8-week old, 230 to 270 g) were purchased from Kiwa Laboratory Animals Co., Ltd. (Wakayama) and used in all experiments. The animals were bred till the experiments in an environment where pellet feeds (MF, Oriental Yeast Co., Ltd., Tokyo) and water can be taken freely in a room kept at 24±2 deg C. All these animal experiments were conducted according to the "Kinki University animal experiment regulation" recognized by Kinki University.

### <Influences of a metal-containing preparation on kinetics in blood of LUFX-EHE in an in-vivo experiment>

### i) Treatment of rats

A cannulation inner tube (Fuji Systems Corporation Ltd., Tokyo) having an internal diameter of 0.5 mm and an outside diameter of 1.0 mm which is used for blood collecting was inserted in the jugular vein of rats on the preceding day before the administration experiment, and the rats were made to abstain from food for 20 hours in an environment where only water can be taken in the animal room.

### ii) Medication method

LVFX (22.5 mg/kg) or LVFX-EHE (25.1 mg/kg) was used independently or together with a polyvalent metal-containing preparation, and was medicated using a gastric tube to animals classified into four groups. The polyvalent metal-containing preparation Al(OH)₃ was administered in a dose of 30 mg/kg subject. As a medication method, the polyvalent metal-containing preparation, LVFX (NQ type antibacterial drug) or LVFX-EHE (hemiacetal-type ester derivative of the NQ type antibacterial drug) was separately ground in a mortar; and after administration of a suspension preparation comprising the polyvalent metal-containing preparation suspended in a 0.5%-Tween20 solution or after administration of a 0.5%-Tween20 solution, NQ type antibacterial drug or its hemiacetal-type ester derivative modulated in 2mL/kg was orally administered successively.

### iii) Blood collecting method

After administration of the drug, a blood sample was collected through a jugular vein cannulation inner tube in an amount of 200 µL each along the time elapsed (after 0, 15, 30, 45, 60, 90, 120, 180, and 240 minutes) in a 1-mL syringe treated with heparin, the blood was centrifuged for 5 minutes at 12,000 rpm and 4 deg C immediately, and 50 µL of a supernatant was taken to make plasma.

### iv) Quantitative determination of LVFX in plasma

The following procedures were carried out successively: addition of 100 µL of methanol solution containing 10 µmol/L of CPFX as an internal standard substance to 50 µL of plasma, then stirring; centrifuging for 5 minutes at 6,200 rpm; sampling 100 µL of the supernatant with a syringe; filtering the sample solution using Millex (registered trademark)-HV (0.45 micrometer, MILLIPORE, Billerica, MA, USA). After that, 10 µL of the filtrate was poured into HPLC, and the concentration of LVFX was measured. The HPLC measurement conditions were as follows; the column used: TSK-GEL ODS-80Ts (250x4.6 mm, TOSOH Corp., Tokyo), the mobile phase used: 20-mm sodium-phosphate buffer solution (pH 3.0) / acetonitrile =79:21 (v/v). The measurement was carried out using Shimazu SPD-20A Spectrometer detector (Shimadzu, Kyoto) with 280 nm detection wavelength, and flow rate was 1.0 mL/min. The retention times obtained by these conditions were 6.9 minutes for ENX, and 7.2 minutes for CPFX.

### v) Statistical analysis

AUC was computed by the trapezoidal method and all experimental results were presented in average +/-standard deviation (S. D.) of each group. The differences of AUCs between 4 groups were computed by performing the multiple comparison by Dunnett's method, and the range of p< 0.05 was understood as having a statistical significance.

### <Influences of a metal-containing preparation on the intestinal absorption of LVFX in in-vitro experiments>

In order to carry out the comparative examination of the actual chelate-forming capability in the intestinal tract of the experimental animal in vitro between the case of independent administration of the prodrug of the present invention and the case of co-administration of the prodrug and a polyvalent metal-containing preparation, the absorption feature and the hydrolysis property in the small intestine brush border membrane were evaluated through absorption properties of the prodrug of the present invention using the intestinal everted sack method (Toshikazu Yamaguchi, Kimiko Yokokawa, Yutaka Sekine and Masahisa Hashimoto, Xenobiotic metabolism and disposition, Vol.6, No.1 (1991)).

### i) Pretreatment of rats

Rats are made to abstain from food for 20 hours in the environment where only water can be taken-in in an animal room on the day preceding the administration experiment.

### ii) Intestinal everted sac technique

### ii-1) Preparation of HEPES buffer isotonic sodium chloride solution

### <Independent administration>

1. Glucose 200 mg was dissolved in 100 ml of 20mM HEPES buffer isotonic sodium chloride solution, and pH is regulated at 7.4 with 5N- or 1N-sodium hydroxide by measuring pH (If pH rises, pH is modulated with IN-hydrochloric acid.). <Co-administration ofAluminum >
1. Similarly in the case of independent administration, glucose 200 mg is dissolved in 100 ml of 20mM HEPES buffer isotonic sodium chloride solution, and pH is regulated at 7.4 with 5N- or 1N-sodium hydroxide by measuring pH (If pH rises, pH is modulated with IN-hydrochloric acid.).
2. AlCl₃ 8.0 mg is dissolved (600 µM).

### ii-2) Anesthetization by pentobarbital (5 w/v%)

1. A SD type male rat of 8-week old after the birth is purchased and undergoes quarantine habituation for two days. After fasting for 24 hours, a pentobarbital sodium (50mg/ml) is injected intraperitoneally in the amount (ml) of: rat weight (g) x 0.05/50 (that is, weight (g) x 0.001).
2. After the rat sleeps completely, it is incised in the abdomen so as to be observed from the stomach till the bladder, and the intestinal tract is removed from the end of the duodenum to the front 5 cm of the cecum.
3. The intestinal tract is cut off every 10 cm carefully, and all portions are washed with physiological saline (both circumference and inside of intestinal).
4. The intestinal tract is made reversed from the stomach side using a stainless steel tube.
5. The cecum side of the reversed intestinal tract is tied with sutures to make bag-shaped.
6. One (1) ml of the HEPES buffer isotonic sodium chloride solution adjusted is put in from the stomach side of the intestinal tract, and the intestinal tract is tied with a suture.
7. The tied intestinal tract is put in the HEPES buffer isotonic sodium chloride solution cooled upon ice, and is preserved.

### ii-3) Preparation of a drug solution

1. LVFX 36mg (or LVFX-M 37mg, LVFX-IHE 40mg, or LVFX-ECH 47mg) is dissolved in 5 ml of DMSO (20mM).
2. One (1) ml of the drug solution is taken, and it is diluted with 9 ml of the HEPES buffer isotonic sodium chloride solution 10 times (2mM).
3. A portion (5 ml) of the solution is further diluted with 95 ml of the HEPES buffer isotonic sodium chloride solution to 20 times (100µM).

### ii-4) Drug absorption

1. Thirty (30) ml of the diluted drug solution is taken to a 50-ml Erlenmeyer flask, and the intestinal tract containing a HEPES buffer isotonic sodium chloride solution is put in.
2. An incubation is carried out for 30 minutes at 37 deg. C with bubbling by mixed oxygen / carbon dioxide in the ratio of O₂/CO₂ =95:5, (Specifically, LVFX or LVFX-EHE is added to a 20mM HEPES buffer isotonic sodium chloride solution to make the whole concentration as 100µM, and the incubation is carried out for 30 or 60 minutes while carrying out a gas bubbling of oxygen and carbon dioxide in the ratio of O₂/CO₂ =95:5. After that, the liquid within the intestinal tract is sampled using a hypodermic needle.
3. The intestinal tract is taken out from the drug solution, and, after flashing and wiping off the drug solution attached to the surroundings of the intestinal tract with the physiological saline, all liquid in the intestinal tract is taken out into the Eppendorf tube.
4. The liquid is centrifuged for 5 minutes at 12,000 rpm.
5. 250 µL of a supernatant liquid is taken as a sample.

### iii) Quantitative determination method

The method is the same as the HPLC measurement conditions mentioned above in-vivo experiment.

### (4) Statistical analysis

All of the experimental results were shown by average +/- standard deviation (S. D.) of each group. The difference between the independent administration and the simultaneous co-administration with aluminum chloride was computed by performing the t-test by the equal dispersion method, and the range of p< 0.05 was understood as having a statistical significance.

### Example 4

The blood concentrations of Levofloxacin in rats were comparatively examined between the case of independent oral administration of Levofloxacin ethoxycarbonyl-1-ethyl hemiacetal-type ester derivative (LVFX-EHE) and the case of oral co-administration with a metal-containing preparation. The obtained results are illustrated in Fig. 1.

Fig. 1 illustrates the blood concentrations of LVFX i) at the time of independent administration of Levofloxacin ethoxycarbonyl-1-ethyl hemiacetal-type ester derivative (LVFX-EHE), ii) at the time of independent administration of Levofloxacin (LVFX), iii) at the time of simultaneous co-administration of LVFX-EHE with a multivalent metal (AlCl₃)-containing antacid, and iv) at the time of simultaneous co-administration of LVFX with a multivalent metal-containing antacid. Significant differences were not observed between i) at the time of independent administration of LVFX-EHE of the present invention, ii) at the time of independent administration of Levofloxacin (LVFX), and iii) at the time of simultaneous co-administration of LUFX-EHE with the multivalent metal-containing antacid of the present invention. Accordingly, it was demonstrated that LVFX-EHE of the present invention acted effectively as a prodrug. In addition, it was also demonstrated that the blood concentration of LVFX was not influenced in LVFX-EHE under concomitant use of polyvalent metal-containing antacid. On the other hand, in the case of co-administration of LVFX and the polyvalent metal-containing antacid, the blood concentration of Levofloxacin decreased significantly. Accordingly, the absorption inhibition by the polyvalent metal-containing antacid was confiimed.

Then, the results of comparison between areas under the plasma drug concentration-time curve (AUCs) calculated for each are illustrated in Fig. 2. Compared with the case of LVFX independent administration, at the time of concomitant use of LVFX and the metal-containing preparation, it turned out that the conversion rate to LVFX falls no less than 50%. That is, inhibition by the metal-containing preparation is significant.

On the other hand, as represented also in Fig. 2, it turns out, by comparison with the independent administration of Levofloxacin (LVFX), that the in-vivo conversion rate of the hemiacetal-type ester derivative (LVFX-EHE) to Levofloxacin is 100%. Moreover, no significant difference is observed between AUC values of LVFX generated in the blood at the time of co-administration of LVFX-EHE and LVFX generated in the blood at the time of LVFX independent administration. Accordingly, it has been confirmed that there is no influence on the blood concentration of LVFX even if LUFX-EHE is administered simultaneously with the metal-containing preparation.

Next, the place in the living body where the administered LVFX-EHE is hydrolyzed and the time required for the hydrolysis were examined.

### Example 5

Hydrolysis of Levofloxacin ethoxycarbonyl-1-ethyl hemiacetal-type ester derivative (LVFX-EHE) to LVFX by the intestinal everted sack method using a small intestine of rat.

The features of hydrolysis of the hemiacetal-type ethyl ester prodrug (LVFX-EHE) of the present invention using a rat intestinal everted sack method in 0.5 hour or 1 hour are represented in Fig. 3.

The leftmost column (a) in Fig. 3 represents the LVFX concentrations in the intestinal lumen at the time of incubating for 30 minutes in the case of independent administration of Levofloxacin (in a broad single-lined frame) and in the case of simultaneous administration of Levofloxacin and the polyvalent metal-containing antacid (AlCl₃) (in a fine double-lined frame). At the time of concomitant use of AlCl₃, the absorption of LVFX decreased by 56%. The reason for this is estimated to be caused by the formation of an insoluble chelate in the outer liquid. On the other hand, the 2nd column (b) from the left shows the LVFX concentrations generated in the intestinal tract at the time of incubating for 30 minutes in the case of the independent administration of LVFX-EHE (in a broad single-lined frame) and in the case of simultaneous administration of LVFX-EHE and AlCl₃ (in a fine double-lined frame). The LVFX concentration in the intestinal lumen generated from LVFX-EHE decreased by 64% as compared to the case of LVFX independent administration. On the other hand, although the decrease was 68% in the case of simultaneous administration of AlCl₃, both decreases do not have a significant difference, and therefore, the influence of the concomitant use of the metal-containing preparation was not observed in the in-situ intestinal tract experiment.

The second column (c) in Fig. 3 represents the LVFX concentrations in the intestinal lumen at the time of incubating for 60 minutes in the case of independent administration of Levofloxacin (in a broad single-lined frame) and in the case of simultaneous administration of Levofloxacin and the polyvalent metal-containing antacid (AlCl₃) (in a fine double-lined frame). The concentration of LVFX decreased by 33% by simultaneous administration of AlCl₃. On the other hand, the Levofloxacin concentration in the intestinal tract at the time of incubating for 60 minutes is represented in the right column (d) in the case of the independent administration of LVFX-EHE (in a broad single-lined frame) and in the case of the simultaneous administration of LVFX-EHE and AlCl₃ (in a double fine-lined frame). Notably, there is no significant difference between the LVFX concentration within the small intestine lumen when LFFX alone was incubated and the LVFX concentration generated within the small intestine lumen when LVFX-EHE was administered alone. This fact demonstrates that LVFX-EHE is completely hydrolyzed into LVFX by the incubation for 60 minutes, and that the region of hydrolysis is a small intestine. Furthermore, since LVFX-EHE blocks the carboxyl group of Levofloxacin with the ethoxycarbonyl 1-ethyl hemiacetal-type ester group, there is also no influence by the simultaneous administration of AlCl₃, and thus, LVFX can be produced almost quantitatively. These facts satisfy our intended concept called "hybrid new quinolones" which do not cause any interactions even if the metal-containing preparation is used simultaneously.

### Example 6

The comparative examination of blood concentrations of Levofloxacin (LVFX) was carried out in the case of independent oral administration of the Levofloxacin isopropoxycarbonyl 1-ethyl hemiacetal-type ester derivative (LVFX-iPrHE) to rats, and in the case of its co-administration to rats with the metal (AlCl₃)-containing preparation. The results obtained are represented in Fig. 4. A significant difference was not found between the case of independent administration of LVFX-iPrHE which is the hemiacetal-type ester derivative of the present invention, the case of independent administration of Levofloxacin (LVFX), and the case of concomitant use of LUFX-iPrHE with the polyvalent metal-containing antacid, as represented in Fig. 4. Accordingly, it was demonstrated that LVFX-EHE of the present invention acted effectively as a prodrug. In addition, it was demonstrated that the blood concentration of LVFX was not influenced in the concomitant use of LVFX-iPrHE with the polyvalent metal-containing antacid. On the other hand, in the case of co-administration of LVFX with the polyvalent metal-containing antacid, the blood concentration of Levofloxacin decreased significantly. Accordingly, the absorption inhibition by the polyvalent metal-containing antacid was confirmed.

Then, the results of comparison of the computed each area under the plasma drug concentration-time curve (AUC) is represented in Fig. 5. Compared with the case of LVFX independent administration, it turns out that in the case of concomitant use of LVFX and the metal-containing preparation, the conversion rate to LVFX decreased no less than 50%. That is, the inhibition by the metal-containing preparation is remarkable.

On the other hand, as can be seen from Fig. 5, the conversion rate of LVFX-iPrHE (which is a hemiacetal-type ester) to Levofloxacin in the living body is 100% as can be seen from comparison with the independent administration of Levofloxacin (LVFX). Moreover, it was confirmed that a concomitant use of LVFX-iPrHE with the metal-containing preparation does not influence the blood concentration, since no significant difference is observed between the AUC of LVFX generated in the blood at the time of concomitant use of LVFX-iPrHE with the metal-containing preparation and the AUC of LVFX at the time of independent administration of LVFX. Then, examinations were carried out as to where in vivo the LVFX-EHE administered is hydrolyzed, and how long the hydrolysis takes a time.

### Example 7

Hydrolysis of Levofloxacin isopropoxycarbonyl-1-ethyl hemiacetal-type ester derivative (LVFX-iPrHE) to LVFX by the intestinal everted sack method using small intestine of rat.

The hydrolysis situation of the hemiacetal-type ethyl ester prodrug (LVFX-iPrHE) of the present invention in 0.5 hour or 1 hour by the rat intestinal everted sack method is represented in Fig. 6.

The leftmost column (a) in Fig. 6 represents the LVFX concentrations in the intestinal lumen at the time of incubating for 30 minutes in the case of independent administration of Levofloxacin (in a broad single-lined frame) and in the case of simultaneous administration of Levofloxacin and the polyvalent metal-containing antacid (AlCl₃) (in a fine double-lined frame). At the time of concomitant use of AlCl₃, the absorption of LVFX decreased by 56%. The reason for this is estimated to be caused by the formation of an insoluble chelate in the outer liquid. On the other hand, the 2nd column (b) from the left shows the LVFX concentrations generated in the intestinal tract at the time of incubating for 30 minutes in the case of the independent administration of LVFX-iPrHE (in a broad single-lined frame) and in the case of simultaneous administration of LVFX-iPrHE and AlCl₃ (in a fine double-lined frame). The LVFX concentration in the intestinal lumen generated from LVFX-iPrHE decreased by 64% as compared with the case of LVFX independent administration. On the other hand, although the decrease was 68% in the case of simultaneous administration of AlCl₃, both decreases do not have a significant difference, and therefore, the influence of the concomitant use of the metal-containing preparation was not observed in the in-situ intestinal tract experiment.

The second column (c) from the right in Fig. 6 represents the LVFX concentrations in the intestinal lumen at the time of incubating for 60 minutes in the case of independent administration of Levofloxacin (in a broad single-lined frame) and in the case of simultaneous administration of Levofloxacin and the polyvalent metal-containing antacid (AlCl₃) (in a fine double-lined frame). The concentration of LVFX decreased by 33% by simultaneous administration of AlCl₃. On the other hand, the Levofloxacin concentration in the intestinal tract at the time of incubating for 60 minutes is represented in the right column (d) in the case of the independent administration of LVFX-iPrHE (in a broad single-lined frame) and in the case of the simultaneous administration of LVFX-iPrHE and AlCl₃ (in a double fine-lined frame). Notably, there is no significant difference between the LVFX concentration within the small intestine lumen when LFFX alone was incubated and the LVFX concentration generated within the small intestine lumen when LVFX-iPrHE was administered alone. This fact demonstrates that LVFX-iPrHE is completely hydrolyzed into LVFX by the incubation for 60 minutes, and that the region of hydrolysis is a small intestine. Furthermore, since LVFX-iPrHE blocks the carboxyl group of Levofloxacin with the ethoxycarbonyl 1-ethyl hemiacetal-type ester group, there is also no influence by the simultaneous administration of AlCl₃, and thus, LVFX can be produced almost quantitatively. These facts satisfy our intended concept called "hybrid new quinolones" which do not cause any interactions even if the metal-containing preparation is used simultaneously.

### Example 8

Comparative experiments of the antibacterial activities between LVFX and LVFX-EHE using Pseudomonas-aeruginosa (ATCC27853), Escherichia coli (ATCC25922 and NIH JC2), and Enterobacter (ZTCC23355).

The MIC activities ("minimum inhibitory concentration") of LVFX and LVFX-EHE to each bacterial strain are represented in Table 11.

**Table 11**

| | Pseudomonasaeruginosa ATCC 27853 | Escherichia coli ATCC 25922 | Escherichia coli NIH JC2 | Enterobacter ATCC 23355 |
|---|---|---|---|---|
| LVFX | 0.5 | ≦0.065 | ≦0.065 | ≦0.065 |
| LVFX-EHE | 8 | 0.5 | 0.5 | 2 |

The MIC activity unit in the table is µg/mL.

Considering the results of Table 11 from clinical break points, the MIC values of LVFX-EHE were non-sensitive to any bacterial strains, since the antibacterial activities of LVFX-EHE were 1/10 or less as compared to those of LVFX. Considering the above results, it was demonstrated that the influences of LVFX-EHE on the normal bacterial flora in intestines were very weak between 30 to 40 minutes from oral administration till absorption of LVFX-EHE. These results can be interpreted as follows: i) LVFX-EHE does, while staying in intestinal tracts, inhibit the enteric-bacterium shift phenomenon which is caused by the strong anti-bactericidal action of LVFX itself, and ii) LVFX-EHE does prevent proliferation of Clostridium-difificile which is a kind of the normal bacterial flora in intestines and has resistances against many antibacterial drugs, and as a result, iii) LVFX-EHE does prevent the toxins produced by the Clostridium-difficile from stimulating the large intestine to result in causing pseudomembranous colitis. That is, it can be estimated that the pyridone carboxyl group of LVFX plays a central role of the antibacterial action, the hemiacetal-type esterification of the pyridone carboxyl group has almost no influence on the intestinal bacterial flora, and as a result, the frequency of occurrence of pseudomembranous colitis also decreases. And it is considered that, once being absorbed from intestinal tracts, the hemiacetal-type ester is hydrolyzed promptly resulting in increase of the blood concentrations of LVFX as represented in Example 3 (Fig. 1 and Fig. 2) and exerts the effective antibacterial activity.

As stated above, it has been demonstrated that the hemiacetal-type ester derivative LVFX-EHE is a hybrid new quinolone which can be co-used simultaneously with the metal-containing antacid and control the onset of pseudomembranous colitis.

### Comparative example 1

Areas under the plasma drug concentration-time curve (AUCs) are represented in Fig. 7 based on the LVFX concentrations produced in the blood when LVFX (22.5 mg/kg) or a simple ester derivative: Levofloxacin isopropyl ester (LVFX-iso: 25.1 mg/kg) obtained in the reference synthesis example 2 was administered to rats orally alone or with aluminum hydroxide (30 mg/kg). Although the AUC was 393.6 µg-min/mL when LVFX was administered alone, the AUC decreased to 196.7 µg-min/mL (about 50% of that in the administration alone) when LVFX was co-administered with Al(OH)₃. On the other hand, with regard to the peak of LVFX derived from LVFX-iso, no peak was detected both at the time of administration alone and co-administration with Al(OH)₃. It turns out that, by forming isopropyl ester derivative, the absorption function is inhibited completely and LVFX-iso does not function at all as a prodrug of LVFX.

### Comparative example 2

In the intestinal everted sack method, the amounts of the intestinal absorption were examined comparatively in the case of LVFX (22.5 mg/kg) alone, in the case of simple ester derivative: LVFX-iso alone obtained in the reference synthesis example 2, and in the case of combined use with aluminum chloride (AlCl₃) (the concentration of the incubation outer liquid in each case is 20mM). The results obtained is represented in Fig. 8. The amounts of the intestinal absorption of Fig. 8 were computed from the buffer volume concentrations in the intestinal tract. Although the amount of intestinal absorption at the time of LVFX administration alone was 4.12 µg/mL as LVFX, it dropped to 2.25 µg/mL (about 54% of the absorption amount at the time of LVFX administration alone) when LVFX and AlCl₃ were administered concomitantly. On the other hand, in the case of LVFX-iso, both in the case of independent administration alone and in the case of concomitant administration with aluminum chloride, the drug substance LVFX as a hydrolysis product is not detected. Accordingly, it was demonstrated that LVFX-iso does not function at all as a prodrug of LVFX.

### Comparative example 3

The blood concentrations (AUC values) of LVFX were comparatively examined based on the blood concentrations of LVFX produced in the blood when LVFX (22.5 mg/kg) or a simple ester derivative: Levofloxacin methyl ester (LVFX-M: 23.5 mg/kg) obtained in the reference synthesis example 1 was administered to rats orally alone or with aluminum hydroxide (30 mg/kg). The results obtained are represented in Fig. 9. Although the AUC was 393.6 µg-hr/mL when LVFX was administered alone, the AUC decreased to 196.7 µg-hr/mL (about 50% of that in administration alone) when LVFX was co-administered with Al(OH)₃. On the other hand, with regard to the peak of LVFX derived from LVFX-M, no peak was detected both at the time of administration alone and co-administration with Al(OH)₃. It turns out that, by forming methyl ester derivative, the absorption function is inhibited completely and LVFX-M does not function at all as a prodrug of LVFX.

### Comparative example 4

In the intestinal everted sack method, the amounts of the intestinal absorption were examined comparatively in the case of LVFX (22.5 mg/kg) alone, in the case of simple ester derivative: LVFX-M alone obtained in the reference synthesis example 1, and in the case of combined use with aluminum chloride (AlCl₃) (the concentration of the incubation outer liquid in each case is 20mM). The results obtained are represented in Fig. 10. The amounts of the intestinal absorption of Fig. 10 were computed from the buffer volume concentrations in the intestinal tract. Although the amount of intestinal absorption at the time of LVFX administration alone was 4.12 µg/mL as LVFX, it dropped to 2.25 µg/mL when LVFX and AlCl₃ were administered concomitantly. On the other hand, in the case of LVFX-M, both in the case of independent administration alone and in the case of concomitant administration with aluminum chloride, the drug substance LVFX as a hydrolysis product was not detected. Accordingly, it was demonstrated that LVFX-M does not function at all as a prodrug of LVFX.

### INDUSTRIAL APPLICABILITY

The alkoxy carbonyl hemiacetal-type ester prodrug of the pyridone carboxylic acid antibacterial drug of the present invention can be used simultaneously and concomitantly with a metal-containing antacid, and can be used as a hybrid new quinolone which inhibits the onset of pseudomembranous colitis.

## Claims

1. An ester prodrug for use for suppression of an enteric-bacterium shift side effect and/or for use in combination with a polyvalent metal-containing medicine, wherein the ester is an alkoxy carbonyl hemiacetal-type ester of a carboxylic acid antibacterial drug represented by the chemical formula (I): 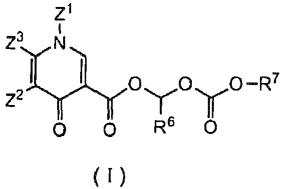
, wherein Z¹-Z³ are the same or different and each is hydrocarbon, nitrogen-containing hydrocarbon, or oxygen-containing hydrocarbon optionally substituted, wherein those adjacent two of Z¹-Z³ may join together to configure a hydrocarbon ring, nitrogen-containing heterocycle, oxygen-containing heterocycle, or nitrogen and oxygen-containing heterocycle optionally substituted,
R⁶ represents H or C₁-C₆ alkyl, and
R⁷ represents C₁-C₆alkyl.

2. The ester prodrug according to Claim 1, wherein the enteric-bacterium shift side effect of the pyridone carboxylic acid antibacterial drug is pseudomembranous colitis.

3. The ester prodrug according to Claim 1 or 2, wherein the polyvalent metal-containing medicine used in combination with an alkoxy carbonyl hemiacetal-tope ester of a polyvalent metal antacid which can be administered orally and substantially simultaneously with the alkoxy carbonyl hemiacetal-type ester prodrug of the pyridone carboxylic acid antibacterial drug.

4. The ester prodrug according to any one of Claims 1-3, wherein the alkoxy carbonyl hemiacetal-type ester of the pyridone carboxylic acid antibacterial drug is represented by the chemical formula (II): 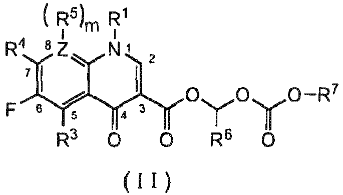 , wherein m represents 1 or 0, R⁶ and R⁷ are the same as above, Z represents C or N, R¹ represents C₁-C₄ alkyl optionally substituted with halogen, C₃-C₆ cycloalkyl optionally substituted with halogen, or phenyl optionally substituted with halogen, R³ represents H, NH₂ or methyl, R⁴ represents 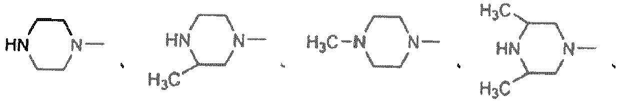 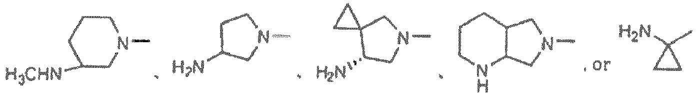 , R⁵ represents H, F, Cl or OCH₃, or R⁵ and R¹ may join together with N of the 1st position or Z of the 8th position to form nitrogen-containing heterocycle or nitrogen and oxygen-containing heterocycle optionally substituted (provided that, when Z=N, m=0).

5. The ester prodrug according to any one of Claims 1-4, wherein, the alkoxy carbonyl hemiacetal-type ester of the pyridone carboxylic acid antibacterial drug of the chemical formula (II) is the alkoxy carbonyl hemiacetal-type ester of the pyridone carboxylic acid antibacterial drug represented by the chemical formula (III): 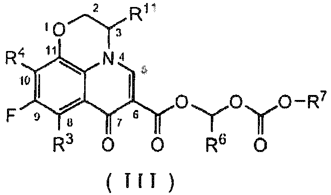 , wherein R³-R⁴ and R⁶-R⁷ are the same as above, and R¹¹ is:

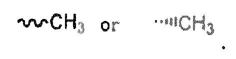

6. The ester prodrug according to any one of Claims 1-4, wherein the ester prodrug corresponds to Z = C and m = 1 in the chemical formula (II) for the alkoxy carbonyl hemiacetal-type ester of the pyridone carboxylic acid antibacterial drug, wherein the ester prodrug is at least one selected from the hemiacetal-type ester derivative of the chemical formula (II-1): 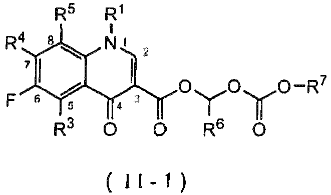 , which has the combination of each substituent R¹, R³, R⁴, R⁵, R⁶, and R⁷, and is selected from:
alkoxy carbonyl hemiacetal-type ester of Norfloxacin, wherein R¹ = ethyl, R³ = H, R⁴ = 1-piperazinyl, R³ = H, R⁶= H or C₁-C₆ alkyl, and R⁷ = C₁-C₆ alkyl;
alkoxy carbonyl hemiacetal-type ester of Lomefloxacin, wherein R¹ = ethyl, R³ = H, R⁴ = 3-methyl-1-piperazinyl, R⁵ = F, R⁶ = H or C₁-C₆ alkyl, and R⁷ =C₁-C₆ alkyl;
akoxy carbonyl hemiacetal-type ester of Fleroxacin, wherein R¹ = CH₂CH₂F, R³ = H, R⁴ = 4-methyl-1-piperazinyl, R⁵ = F, R⁶ = H or C₁-C₆ alkyl, and R⁷ = C₁-C₆ alkyl;
alkoxy carbonyl hemiacetal-type ester of Ciprofloxacin, wherein R¹ = cyclopropyl, R³ = H, R⁴ = 1-piperazinyl, R⁵ = H, R⁶= H or C₁-C₆ alkyl, and R⁷ = C₁-C₆ alkyl;
alkoxy carbonyl hemiacetal-type ester of Sparfloxacin, wherein R¹ = cyclopropyl, R³=NH₂, R⁴=3, 5- dimethyl- 1-piperazinyl, R⁵ =F, R⁶=H or C₁-C₆ alkyl, and R⁷ =C₁-C₆ alkyl;
alkoxy carbonyl hemiacetal-type ester of Grepafloxacin wherein R¹ = cyclopropyl, R³=CH₃, R⁴ =3- methyl-1-piperazinyl, R⁵=H, R⁶=H or C₁-C₆ alkyl, and R⁷ =C₁-C₆ alkyl;
alkoxy carbonyl hemiacetal-type ester of Balofloxacin, wherein R¹ = cyclopropyl, R³=H, R⁴ =3-methylamino-1-piperidino, R⁵=OCH₃, R⁶ =H or C₁-C₆ alkyl, and R⁷ =C₁-C₆ alkyl;
alkoxy carbonyl hemiacetal-type ester of Temafloxacin, wherein R¹=2,4-difluorophenyl, R³=H, R⁴ =3-methyl-1-piperazinyl, R⁵=H, R⁶=H or C₁-C₆ alkyl, and R⁷ =C₁-C₆ alkyl; and
alkoxy carbonyl hemiacetal-type ester of Sitafloxacin, wherein
R¹ = 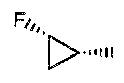
,R³=H,
R⁴ = 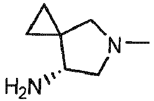
, R⁵ =Cl, R⁶ =H or C₁-C₆ alkyl, and R⁷ =C₁-C₆ alkyl.

7. The ester prodrug according to any one of Claims 1-5 which has the combination of each substituent R¹¹, R³, R⁴, R⁶, and R⁷ in alkoxy carbonyl hemiacetal-type ester of the pyridone carboxylic acid antibacterial drug represented by the chemical formula (III) and is at least one alkoxy carbonyl hemiacetal-type ester of the pyridone carboxylic acid antibacterial drug selected from the followings:
alkoxy carbonyl hemiacetal-type ester of Ofloxacin, wherein R¹¹ = methyl, R³=H, R⁴ =4-methyl-1-piperazinyl, R⁶ =H or C₁-C₆ alkyl, and R⁷ =C₁-C₆, alkyl,
alkoxy carbonyl hemiacetal-type ester of Levofloxacin, wherein R¹¹ = 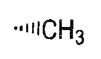
, R³=H, R⁴ -4-methyl-1-piperazinyl, R⁶ =H or C₁-C₆ alkyl, and R⁷ =C₁-C₆ alkyl, and
alkoxy carbonyl hemiacetal-type ester of the Pazufloxacin, wherein R¹¹ = 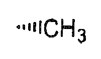
, R³=H, R⁴ -1-amino-1-cyclopropyl, R⁶=H or C₁-C₆ alkyl, and R⁷ =C₁-C₆ alkyl.

8. The ester prodrug according to any one of Claims 1-5 and 7, wherein alkoxy carbonyl hemiacetal-type ester of the pyridone carboxylic acid antibacterial drug of the chemical formula (III) is alkoxy carbonyl hemiacetal-type ester of Levofloxacin represented by the chemical formula (IV): 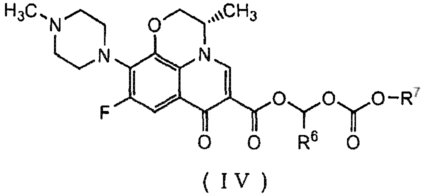 , wherein R⁶ and R⁷ are the same as mentioned as above.

9. The ester prodrug according to any one of Claims 1-5, 7 and 8, wherein alkoxy carbonyl hemiacetal-type ester of the pyridone carboxylic acid antibacterial drug of the chemical formula (III) is ethoxycarbonyl-1-ethyl hemiacetal-type ester of Levofloxacin represented by the chemical formula (V): 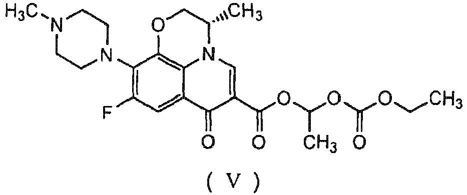 , or isopropoxycarbonyl-1-ethyl hemiacetal-type ester of the Levofloxacin represented by the chemical formula (VI):

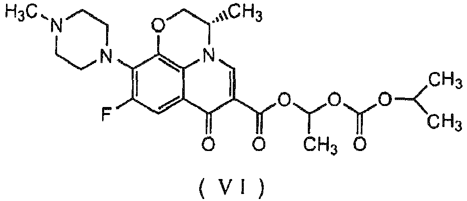

10. The ester prodrug according to any one of Claims 1-4, wherein the ester prodrug corresponds to Z = N and m = 0 in the chemical formula (II) for the alkoxy carbonyl hemiacetal-type ester of the pyridone carboxylic acid antibacterial drug, wherein the ester prodrug is selected from the hemiacetal-type ester derivative of the chemical formula (VII): 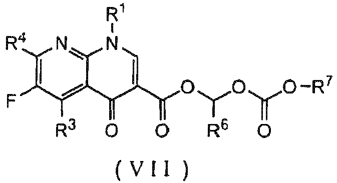
, wherein R¹ = ethyl, R³=H, R⁴ =1-piperazinyl, R⁶=H or C₁-C₆ alkyl, and R⁷ =C₁-C₆ alkyl: which is alkoxy carbonyl hemiacetal-type ester of Enoxacin, or
R¹ =2,4-difluorophenyl, R³=H, R⁴ =3-amino-1-pyrrolidinyl, R⁶=H or C ₁-C₆ alkyl, and
R⁷ =C₁-C₆ alkyl: which is alkoxy carbonyl hemiacetal-type ester of Tosufloxacin.

11. The ester prodrug according to any one of Claims 1-4 and 10, wherein the alkoxy carbonyl hemiacetal-type ester of Enoxacin is ethoxycarbonyl 1-ethyl hemiacetal-type ester of Enoxacin represented by the chemical formula (VIII): 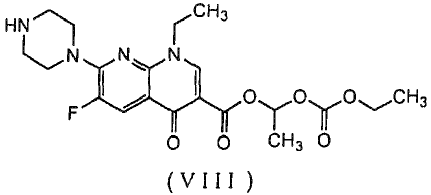 , or isopropoxycarbonyl 1-ethyl hemiacetal-type ester of Enoxacin represented by the chemical formula (IX):

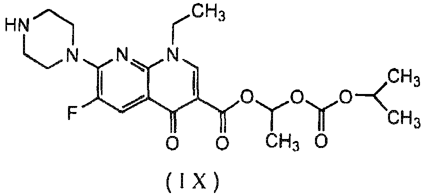

12. The ester prodrug according to any one of Claims 1-4, and 10, wherein
the alkoxy carbonyl hemiacetal-type ester prodrug of Tosufloxacin is ethoxycarbonyl 1-ethyl hemiacetal-type ester prodrug of Tosufloxacin represented by the chemical formula (X): 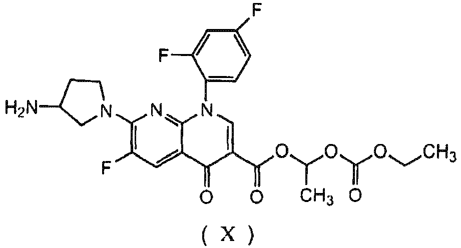 , or isopropoxycarbonyl-1-ethyl hemiacetal-type ester of Tosufloxacin represented by the chemical formula (XI):

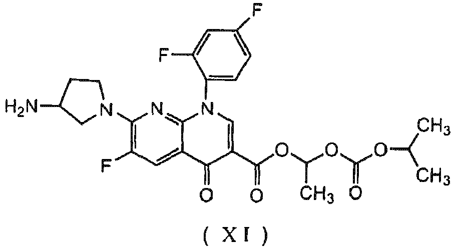

13. A pharmaceutical composition for oral administration comprising alkoxy carbonyl hemiacetal-type ester prodrug of the pyridone carboxylic acid antibacterial drug according to any one of Claims 1-12 and excipients.

14. The pharmaceutical composition according to Claim 13 for administering orally and substantially simultaneously with a polyvalent metal-containing medicine.

15. The pharmaceutical composition according to Claim 14, wherein the polyvalent metal-containing medicine is a polyvalent metal-containing antacid.

16. A kit consisting of a pharmaceutical composition according to Claim 14 or 15 and a polyvalent metal-containing medicine.

17. The kit according to Claim 16, wherein the polyvalent metal-containing medicine is a polyvalent metal-containing antacid.

18. An alkoxy carbonyl hemiacetal-type ester compound of the pyridone carboxylic acid antibacterial drug or a pharmaceutically acceptable salt thereof, wherein the ester compound is represented by the chemical formula (I): 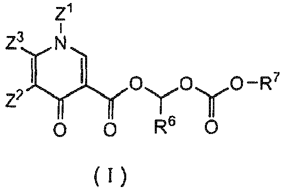 , wherein each of Z¹-Z³ represents hydrocarbon, nitrogen-containing hydrocarbon, or oxygen-containing hydrocarbon optionally substituted, wherein those adjacent two of them may join together to configure a hydrocarbon ring, nitrogen-containing heterocycle. oxygen-containing heterocycle, or nitrogen- and oxygen-containing heterocycle optionally substituted, R⁶ represents H or C₁-C₆ alkyl, and R⁷ represents C₁-C₆ alkyl.

19. The alkoxy carbonyl hemiacetal-type ester compound of the pyridone carboxylic acid antibacterial drug according to Claim 18, wherein the ester compound is represented by the chemical formula (II): 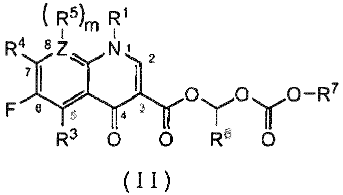
, wherein, m represents 1 or 0, R⁶ and R⁷ are the same as above, Z represents C or N, R¹ represents C₁-C₄ alkyl optionally substituted with halogen, C₃-C₆ cycloalkyl optionally substituted with halogen, or phenyl optionally substituted with halogen, R³ represents H, NH₂, or methyl, R⁴ represents: 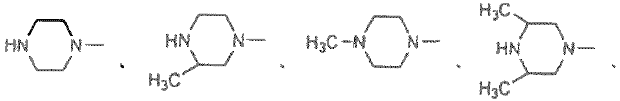 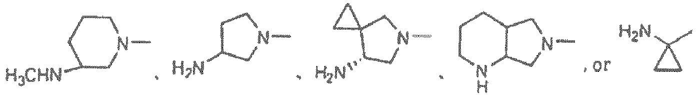
, R⁵ represents H, F, Cl, or OCH₃, or
R⁵ and R¹ may join together with N of the 1st position and Z of the 8th position to configure nitrogen-containing heterocycle, or nitrogen and oxygen-containing heterocycle optionally substituted (provided that; m = 0 when Z = N),
or when Z = C, R¹ and R⁵ join together with N atom and C atom of the quinolone ring to configure a cyclic compound resulting in the formation of alkoxy carbonyl hemiacetal-type ester of an oxazine-containing pyridone carboxylic acid antibacterial drug represented by the chemical formula (III): 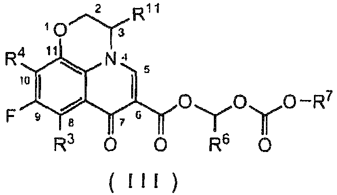 , wherein R³-R⁴ and R⁶-R⁷ are the same as above, and R¹¹ is:

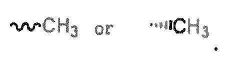

20. The alkoxy carbonyl hemiacetal-type ester compound according to Claim 18 or 19, wherein the ester compound corresponds to Z = C and m = 1 in the chemical formula (II) for the alkoxy carbonyl hemiacetal-type ester of the pyridone carboxylic acid antibacterial drug, wherein the ester compound is selected from the hemiacetal-type ester derivative of the chemical formula (II-1): 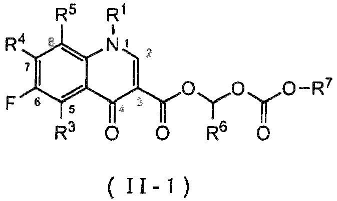 , which is the combination of each substituent R¹, R³, R⁴, R⁵, R⁶, and R⁷, and is selected from:
alkoxy carbonyl hemiacetal-type ester of Norfloxacin, wherein R¹ = ethyl, R³ = H, R⁴ = 1-piperazinyl, R⁵ = H, R⁶ = H or C₁-C₆ alkyl, and R⁷ = C₁-C₆ alkyl;
alkoxy carbonyl hemiacetal-type ester of Lomefloxacin, wherein R¹ = ethyl, R³ = H, R⁴ = 3-methyl-1-piperazinyl, R⁵ = F, R⁶ = H or C₁-C₆ alkyl, and R⁷ = C₁-C₆ alkyl;
alkoxy carbonyl hemiacetal-type ester of Fleroxacin, wherein R¹ = CH₂CH₂F, R³ = H, R⁴ = 4-methyl-1-piperazinyl, R⁵ = F, R⁶ H or C₁-C₆ alkyl, and R⁷ = C₁-C₆ alkyl;
alkoxy carbonyl hemiacetal-type ester of Ciprofloxacin, wherein R¹ = cyclopropyl, R³ = H, R⁴ = 1-piperazinyl, R⁵ = H, R⁶ = H or C₁-C₆ alkyl, and R⁷ = C₁-C₆ alkyl;
alkoxy carbonyl hemiacetal-type ester of Sparfloxacin, wherein R¹ = cyclopropyl, R³=NH₂, R⁴=3, 5- dimethyl- 1-piperazinyl, R⁵ =F, R⁶ =H or C ₁-C₆ alkyl, and R⁷ =C₁-C₆ alkyl;
alkoxy carbonyl hemiacetal-type ester of Grepafloxacin, wherein R¹ = cyclopropyl, R³=CH₃, R⁴ =3- methyl-1-piperazinyl, R⁵=H, R⁶=H or C₁-C₆ alkyl, and R⁷ =C₁-C₆ alkyl;
alkoxy carbonyl hemiacetal-type ester of Balofloxacin, wherein R¹ = cyclopropyl, R³=H, R⁴ = 3-methylamino-1-piperidino, R⁵=OCH₃, R⁶ =H or C₁-C₆ alkyl, and R⁷ -C₁-C₆ alkyl;
alkoxy carbonyl hemiacetal-type ester of Temafloxacin, wherein R¹=2,4-difluorophenyl, R³=H, R⁴ =3-methyl-1-piperazinyl, R⁵=H, R⁶=H or C₁-C₆ alkyl, and R⁷ =C₁-C₆ alkyl; and
alkoxy carbonyl hemiacetal-type ester of Sitafloxacin, wherein
R¹ = 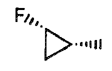
, R³=H,
R⁴ = 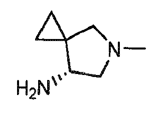
, R⁵=Cl, R⁶=H or C ₁-C₆ alkyl, and R⁷ =C₁-C₆ alkyl.

21. The alkoxy carbonyl hemiacetal-type ester compound according to Claim 18 or 19, wherein the combination of each substituent R¹¹, R³, R⁴, R⁶, and R⁷ in alkoxy carbonyl hemiacetal-type ester of the pyridone carboxylic acid antibacterial drug represented by the chemical formula (III) is at least one alkoxy carbonyl hemiacetal-type ester of the pyridone carboxylic acid antibacterial drug selected from the followings:
alkoxy carbonyl hemiacetal-type ester of Ofloxacin, wherein R¹¹ = methyl, R³=H, R⁴ =4-methyl-1-piperazinyl, R⁶=H or C₁-C₆ alkyl, and R⁷ =C₁-C₆ alkyl,
alkoxy carbonyl hemiacetal-type ester of Levofloxacin, wherein R¹¹ = 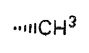 , R³=H, R⁴ =4-methyl-1-piperazinyl, R⁶=H or C₁-C₆ alkyl, and R⁷ =C₁-C₆ alkyl, and
alkoxy carbonyl hemiacetal-type ester of the Pazufloxacin, wherein R¹¹ = 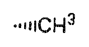 , R³=H, R⁴ =1-amino-1-cyclopropyl, R⁶=H or C ₁-C₆ alkyl, and R⁷ =C₁-C₆ alkyl.

22. The alkoxy carbonyl hemiacetal-type ester compound according to Claim 18 or 19, wherein the ester compound corresponds to Z = N and m = 0 in the chemical formula (II) for the alkoxy carbonyl hemiacetal-type ester of the pyridone carboxylic acid antibacterial drug, wherein the ester compound is selected from the hemiacetal-type ester derivative of the chemical formula (VII): 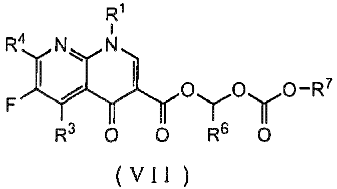
, wherein R¹ = ethyl, R³=H, R⁴ =1-piperazinyl, R⁶ =H or C₁-C₆ alkyl, and R⁷ =C₁-C₆ alkyl: which is alkoxy carbonyl hemiacetal-type ester of Enoxacin, or
R¹ =2,4-difluorophenyl, R³=H, R⁴ =3-amino-1-pyrrolidinyl, R⁶ =H or C ₁-C₆ alkyl, and
R⁷ =C₁-C₆ alkyl: which is alkoxy carbonyl hemiacetal-type ester of Tosufloxacin.

23. The alkoxy carbonyl hemiacetal-type ester compound according to any one of Claims 18-22, wherein the alkoxy carbonyl hemiacetal-type ester compound of the pyridone carboxylic acid antibacterial drug is at least one alkoxy carbonyl hemiacetal-type ester selected from: ethoxycarbonyl 1-ethyl hemiacetal-type ester of Levofloxacin represented by the chemical formula (V): 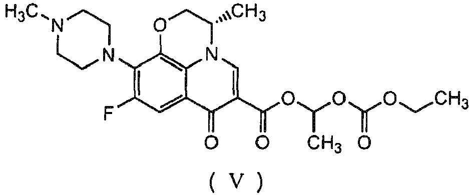 , isopropoxycarbonyl-1-ethyl hemiacetal-type ester of Levofloxacin represented by the chemical formula (VI): 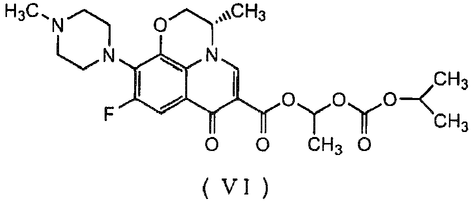 , ethoxycarbonyl 1-ethyl hemiacetal-type ester of Enoxacin represented by the chemical formula (VIII): 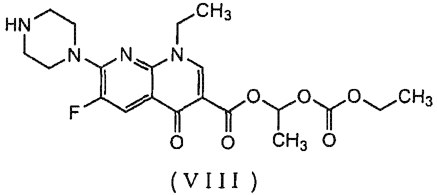 , isopropoxycarbonyl-1-ethyl hemiacetal-type ester of Enoxacin represented by the chemical formula (IX): 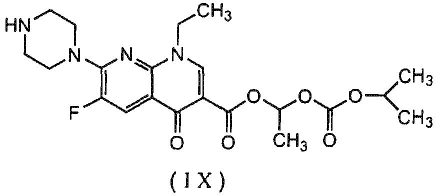 , ethoxycarbonyl 1-ethyl hemiacetal-type ester of Tosufloxacin represented by the chemical formula (X): 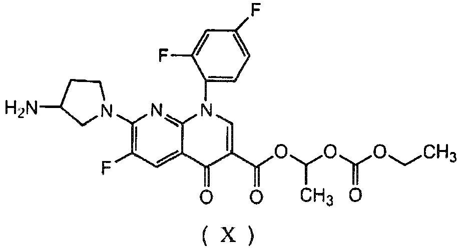 , and isopropoxycarbonyl-1-ethyl hemiacetal-type ester of Tosulloxacin represented by the chemical formula (XI):

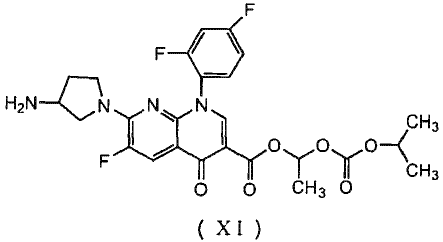

24. A method for producing an alkoxy carbonyl hemiacetal-type ester of the pyridone carboxylic acid antibacterial drug comprising reacting pyridone carboxylic acid represented by the chemical formula (I-1) or its salt: 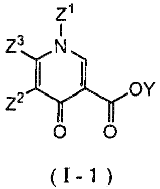 , wherein Z¹-Z³ are the same as above, and Y is H, alkali metal, alkaline earth metal or organic base, with alpha-halogenized dialkyl carbonate represented by the chemical formula (XII): 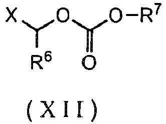 , wherein R⁶ and R⁷ are the same as above, and X is halogen selected from Cl, Br, and I, to produce the alkoxy carbonyl hemiacetal-type ester of pyridone carboxylic acid antibacterial drug represented by chemical formula (I): 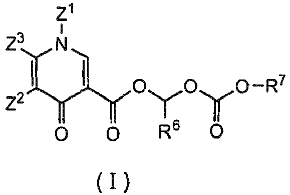 , wherein Z¹-Z³, R⁶, and R⁷ are the same as above.

25. The method for producing the alkoxy carbonyl hemiacetal-type ester according to Claim 24, wherein the pyridone carboxylic acid or its salt is pyridone carboxylic acid represented by the chemical formula (II-A) or its salt: 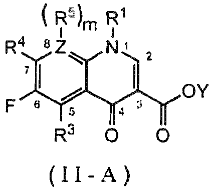
, wherein m represent 1 or 0,
R¹ is C₁-C₄ alkyl optionally substituted with halogen, C₃-C₆ cycloalkyl optionally substituted with halogen, or phenyl optionally substituted with halogen,
R³ represents H, NH², or methyl,
R⁴ represents: 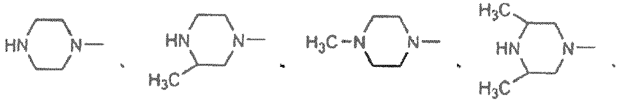 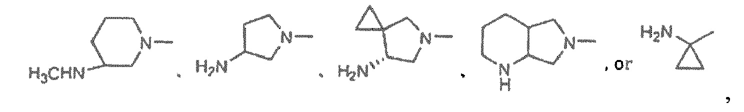
R⁵ represents H, F, Cl, or OCH₃, or
R⁵ and R¹ may join together with N of the 1st position and Z of the 8th position to form a nitrogen-containing heterocycle, or a nitrogen and oxygen-containing heterocycle optionally substituted (provided that; when Z = N, m = 0), Y is H, alkali metal, alkaline earth metal or organic base, and
, the ester obtained is alkoxy carbonyl hemiacetal-type ester of the pyridone carboxylic acid antibacterial drug represented by the chemical formula (II): 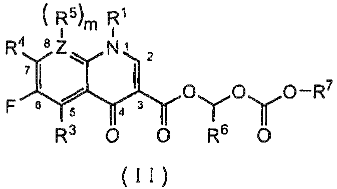 , wherein m, R¹, R³-R⁷, and Z are the same as above.

26. The method for producing the alkoxy carbonyl hemiacetal-type ester according to Claim 24, wherein the pyridone carboxylic acid or its salt is pyridone carboxylic acid represented by the chemical formula (III-1) or the salt thereof: 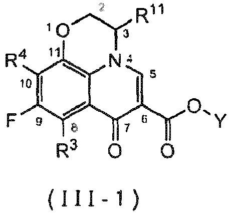
, wherein R³ represents H, NH₂ or methyl, R⁴ represents: 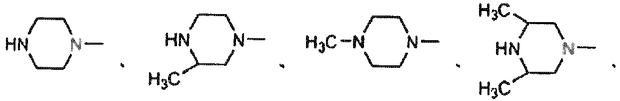 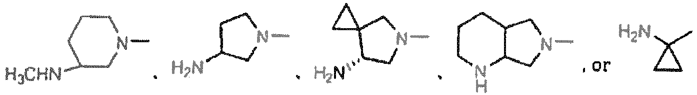
, Y is H, alkali metal, alkaline earth metal or organic base, R¹¹ is 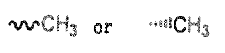
, and the ester obtained is the alkoxy carbonyl hemiacetal-type ester of the pyridone carboxylic acid antibacterial drug represented by the chemical formula (III): 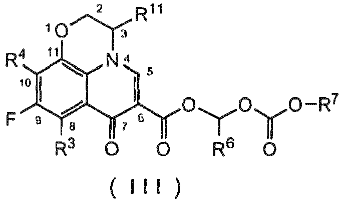 , wherein R³-R⁴, R⁶-R⁷, and R¹¹ are the same as above.

27. The method for producing the alkoxy carbonyl hemiacetal-type ester according to any one of Claims 24-26, wherein NaI is made to coexist together when X=Cl or Br in the alpha-halogenized dialkyl carbonate of the chemical formula (XII).

28. The method for producing the alkoxy carbonyl hemiacetal-type ester according to any one of Claims 24-27, wherein the method is carried out in the presence of at least one base selected from inorganic base or organic base.

29. The method for producing the alkoxy carbonyl hemiacetal-type ester according to any one of Claims 25-28, wherein the pyridone carboxylic acid or its salt is represented by the chemical formula (II-A): 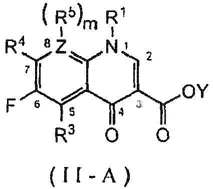 , wherein R¹, R³, R⁴, R⁵, Z, m, and Y are the same as above, and further at least one of substituents R¹ and R³-R⁵ is a substituent comprising an active amino group, wherein the method is for producing an alkoxy carbonyl hemiacetal-type ester represented by the chemical formula (II): 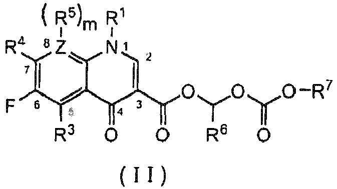 , wherein m, R¹, R³-R⁷, and Z are the same as above,
, wherein the method is **characterized by** comprising the sequential steps of:
(i) protecting the active amino group by benzyloxycarbonyl,
(ii) reacting alpha-halogenized dialkyl carbonate represented by the chemical formula (XII) to obtain a hemiacetal-type ester derivative, and
(iii) removing the benzyloxycarbonyl protecting group by catalytic hydrogenation decomposition.

30. The method for producing the alkoxy carbonyl hemiacetal-type ester according to Claim 29, wherein the active amino group is cyclic secondary amine.

31. The method for producing the alkoxy carbonyl hemiacetal-type ester according to Claim 30, wherein the cyclic secondary amine is at least one cyclic secondary amine selected from the following chemical formula:
